# EUROPEAN PATENT SPECIFICATION

(11) **EP 3 687 556 B1**
(45) Date of publication and mention of the grant of the patent: **30.04.2025**
(21) Application number: 18788679.1
(22) Date of filing: 01.10.2018
(51) Int. Cl.: A61K 35/768, C12N 7/08

(54) **METHOD FOR TREATING COLORECTAL CANCER WITH A COXSACKIEVIRUS B3 (CVB3) VARIANT**
VERFAHREN ZUR BEHANDLUNG VON KOLOREKTALKREBS MIT EINER COXSACKIEVIRUS B3(CVB3)-VARIANTE
MÉTHODE DE TRAITEMENT DU CANCER COLORECTAL AVEC UN VARIANT DU VIRUS COXSACKIE B3 (CVB3)

(30) Priority: 29.09.2017 US 201715720850
(43) Date of publication of application: 05.08.2020
(73) Proprietor: Technische Universität Berlin, 10623 Berlin (DE)
(72) Inventor: FECHNER, Henry, 15926 Luckau (DE); HAZINI, Ahmet, 13353 Berlin (DE); WOLLNY, Vanessa, 12103 Berlin (DE)
(74) Representative: Fleuchaus & Gallo Partnerschaft mbB
(86) International application number: PCT/EP2018/076624
(87) International publication number: WO 2019/063838

(56) References cited:
- YANG JIA ET AL: "Extremely Low Organ Toxicity and Strong Antitumor Activity of miR-34-Regulated Oncolytic Coxsackievirus B3", MOLECULAR THERAPY - ONCOLYTICS, vol. 12, 1 March 2019 (2019-03-01), pages 246 - 258, XP055614324, ISSN: 2372-7705, DOI: 10.1016/j.omto.2019.01.003
- BEIBEI WANG ET AL: "A Novel Combination Therapy for Human Oxaliplatin-resistant Colorectal Cancer Using Oxaliplatin and Coxsackievirus A11", ANTICANCER RESEARCH, vol. 38, no. 11, 1 November 2018 (2018-11-01), GR, pages 6121 - 6126, XP055614325, ISSN: 0250-7005, DOI: 10.21873/anticanres.12963
- AHMET HAZINI ET AL: "Heparan Sulfate Binding Coxsackievirus B3 Strain PD: A Novel Avirulent Oncolytic Agent Against Human Colorectal Carcinoma", HUMAN GENE THERAPY, vol. 29, no. 11, 1 November 2018 (2018-11-01), US, pages 1301 - 1314, XP055536199, ISSN: 1043-0342, DOI: 10.1089/hum.2018.036
- A. E. ZAUTNER ET AL: "Heparan Sulfates and Coxsackievirus-Adenovirus Receptor: Each One Mediates Coxsackievirus B3 PD Infection", JOURNAL OF VIROLOGY., vol. 77, no. 18, 15 September 2003 (2003-09-15), US, pages 10071 - 10077, XP055536200, ISSN: 0022-538X, DOI: 10.1128/JVI.77.18.10071-10077.2003
- A. E. ZAUTNER ET AL: "N- and 6-O-Sulfated Heparan Sulfates Mediate Internalization of Coxsackievirus B3 Variant PD into CHO-K1 Cells", JOURNAL OF VIROLOGY., vol. 80, no. 13, 14 June 2006 (2006-06-14), US, pages 6629 - 6636, XP055536203, ISSN: 0022-538X, DOI: 10.1128/JVI.01988-05
- SCHMIDTKE M ET AL: "Attachment of Coxsackievirus B3 Variants to Various Cell Lines: Mapping of Phenotypic Differences to Capsid Protein VP1", VIROLOGY, ELSEVIER, AMSTERDAM, NL, vol. 275, no. 1, 15 September 2000 (2000-09-15), pages 77 - 88, XP004435910, ISSN: 0042-6822, DOI: 10.1006/VIRO.2000.0485
- SCHMIDTKE M ET AL: "The viral genetic background determines the outcome of coxsackievirus b3 infection in outbred NMRI mice", JOURNAL OF MEDICAL VIROLOGY, vol. 79, no. 9, September 2007 (2007-09-01), pages 1334 - 1342, XP002787564, ISSN: 0146-6615
- ERIK H. KNELSON ET AL: "Heparan sulfate signaling in cancer", TRENDS IN BIOCHEMICAL SCIENCES, vol. 39, no. 6, 1 June 2014 (2014-06-01), AMSTERDAM, NL, pages 277 - 288, XP055536387, ISSN: 0968-0004, DOI: 10.1016/j.tibs.2014.03.001
- S. MIYAMOTO ET AL: "Coxsackievirus B3 Is an Oncolytic Virus with Immunostimulatory Properties That Is Active against Lung Adenocarcinoma", CANCER RESEARCH, vol. 72, no. 10, 29 March 2012 (2012-03-29), US, pages 2609 - 2621, XP055536229, ISSN: 0008-5472, DOI: 10.1158/0008-5472.CAN-11-3185

## Description

### Field of invention

The invention relates to Coxsackievirus B3 group (CVB3) viruses and its derivatives for the use in medicine and particularly for treating subjects having cancer, which expresses a heparan sulphate surface receptor on the cancer cells.

### Background of the invention

Virotherapy with oncolytic viruses (OV) is a new form of therapy for the treatment of malignant tumor diseases, which has demonstrated its effectiveness in several clinical trials in the last two decades (Fukuhara et al., 2016). The anti-tumor effect is based on a dual mechanism. One is the destruction of the tumor cells by the tumor cell-specific replication of the OV, wherein seemingly OVs do selectively replicate in cancer cells, spread within tumor tissue and lead to tumor destruction. On the other hand due to the viral replication immunological processes are induced, which leads to a systemic anti-tumoral immune response (Kaufman et al., review 2015). Various DNA viruses, such as adenovirus, parvovirus, vaccinia virus and herpesvirus, and RNA viruses, such as Coxsackie-A-virus (CVA), vesicular stomatitis virus and reoviruses have been used as OV for treatment of cancer.

RNA viruses have a short replication cycle and produce a very large number of progeny, which gives them an advantage relative to DNA viruses in oncolytic virotherapy. Moreover, RNA viruses may be safer, as they lack the potential for genotoxic effects caused by integration into the host genome. In the course of the last fifteen years, enteroviruses such as CVA21, echovirus 1 and poliovirus, which are single-stranded RNA-viruses and belong to the *Picornaviridae* family, were evaluated for their potential as oncolytic agents against melanoma, breast and prostate cancer (Berry et al., 2008; Shafren et al., 2004; Skelding et al., 2009). Poliovirus and Coxsackivirus of strains A and B from the genus Enterovirus (EV) of the family *Picornaviridae* have been considered as promising OV for the therapy of tumor diseases.

Recently, another member of this group, Coxsackievirus B3 group (CVB3), strain Nancy, was described as a novel OV for treatment of lung carcinomas. The most comprehensive studies concerning effectiveness so far have been conducted with the oncolytic poliovirus variant PVS-RIPO in glioblastomas and Coxsackievirus A21 (CVA21) in melanomas. Of the Coxsackie B viruses (CVB), only CVB3 Nancy as OV in lung carcinomas was comprehensively described in an *in vivo* mouse model (Miyamoto et al., 2012). There it was shown that wild-type CVB3 (Nancy) can lyse human lung carcinoma cell tumors in nude mice. At the same time, the authors showed that although CVB3 (Nancy) induced inflammatory side effects, they were not considered as severe and did not lead to the death of the animals. No treatment-related mortality, but moderate hepatic dysfunction and mild myocarditis were reported to be the main side effects of CVB3 Nancy treatment of lung carcinoma in mice.

A number of CVB3 strains have been characterized by their tissue tropism and organ toxicity in order to better understand virus-host interaction and pathogenesis caused by viral infection. Among these CVB3 strains, there are strains which are highly cardiotropic, such as CVB3 H3, 31-1-31, M2, HA or H310A1, whereas a number of other strains have been found to be low or non-cardiotropic, e.g. Nancy and PD. There are also CVB3 strains that preferentially infect the liver. Moreover, almost all known CVB3 strains are able to infect the pancreas.

The difference in pathogenicity is believed to be attributable to viral capsid proteins, which are directly involved in virus-cell attachment and virus uptake. In general, most of CVB3 strains utilize the Coxsackievirus and adenovirus receptor (CAR) as primary and the decay accelerating factor (DAF) as co-receptors to infect cells (Bergelson et al., 1998; Bergelson et al., 1995). However, recently it was found that the strain CVB3 PD has an additional and unique receptor tropism as it can use heparan sulfates (HS) to enter the cells (Zautner et al., 2003).

Furthermore, as it was previously shown that CVB3 Nancy induces severe inflammation of the pancreas and the heart in mice (Stein et al., 2015; Pinkert et al., 2009) and in addition, that some CVBs are associated with the development of inflammatory and dilated cardiomyopathies in humans (Andreoletti et al., 2009; Andreoletti et al., 2007) or that CVB3 (Nancy) has been described in connection with severe infections of children (Ronellenfitsch et al., 2014), it seemed questionable to what extent CVB3 Nancy could actually be considered a safe OV.

It is therefore an object of the present invention to overcome the described disadvantages of the state of the art OVs in the treatment of cancer, especially with respect to efficiency and safety of used OVs. Moreover, it is the aim of the present invention to provide a new variant of CVB3 with substantially improved oncolytic efficiency and safety compared to known strains such as e.g. CVB3 strain Nancy.

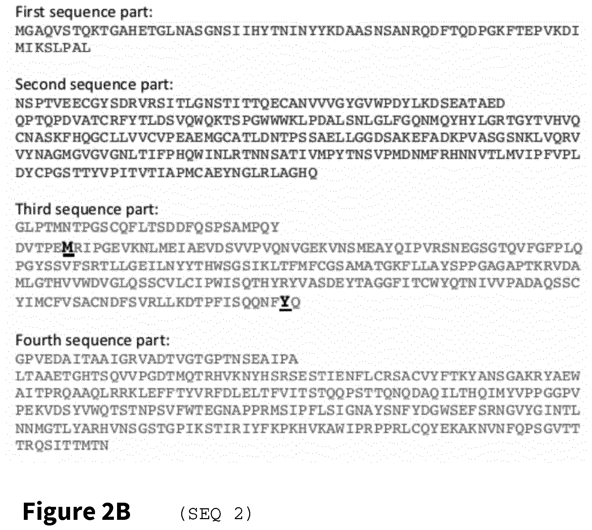

**Summary of the invention** The present invention provides a PD variant of the coxsackie B3 group (CVB3) virus for use in the treatment of cancer, wherein the PD variant is defined by an amino acid sequence comprising the sequence of figure 2B (SEQ 2) and wherein the cancer is colorectal carcinoma.

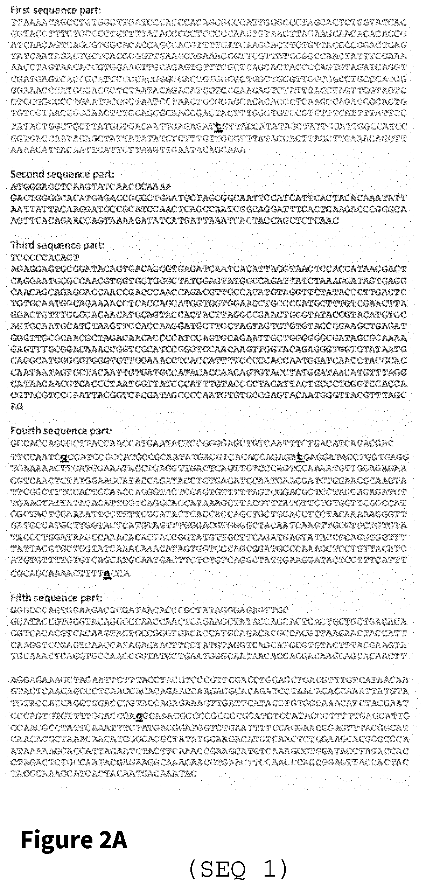

The PD variant for use in the treatment of colorectal carcinoma mentioned above is also defined by the nucleotide sequence comprising the sequence of figure 2A (SEQ 1).

The PD variant for use in the treatment of colorectal carcinoma can be used in a range between about 5x10⁵ and about 3×10⁸ plaque forming units (PFU).

The invention is defined by the claims and any other aspects, configurations or embodiments set forth herein not falling within the scope of the claims are for information only. Any references in the description to methods of treatment refer to the compounds, pharmaceutical compositions and medicaments of the present invention for use in a method for treatment of the human (or animal) body by therapy or for diagnosis.

### Detailed description of the invention

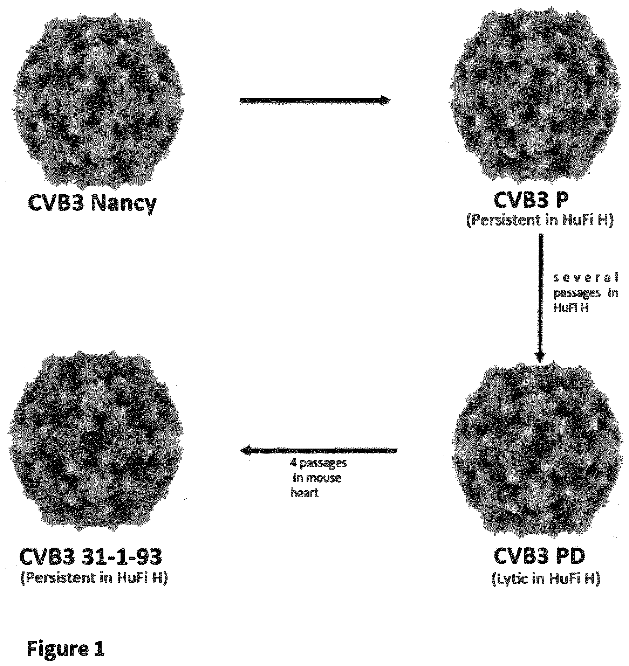

To improve the host range and utilize the unique receptor tropism a novel CVB3 PD variant was generated from the pre-described CVB3 PD by passaging. For this, the pre-described CVB3 variants were treated by serial passaging in specific target cells *in vitro* and in tissues *in vivo* **(****Figure 1****).** Thus, a modified CVB3 variant PD, which is in the following and in the examples regularly addressed as PD-0, could be isolated after 13-fold passaging of the CVB3 variant CVB3 P in human fetal primary fibroblast cells H (HuFi H). In contrast to the CVB3 P variant, which induces a persistent CVB3 infection in HuFi H, PD-0 was able to completely lyse HuFi H cell cultures (**Figure 1**).

It has been shown that in the newly generated CVB3 PD-0 variant four amino acid (AA) substitutions were detected in the N-terminal area of the VP1 domain. It is believed that due to at least one or two of these mutations, PD-0 can use heparan sulfates at the cell surface in addition to the CVB3 receptors CAR and DAF for incorporation into the cell. This is particularly important as the CAR is known to be the primary CVB3 receptor responsible for the infection of tumor cells and which expression is regarded to be low on some tumor cells (Fechner et al., 2000).

Accordingly, the selection of the new CVB3 variant and the identification of sequence alterations, which lead to the use of alternative receptors and, thus, alternative host ranges, provides substantial improvements in the inventive method of treating cancer, wherein the cancer comprises cells expressing heparin sulfates on its cell surface.

According to one embodiment of the invention the CVB3 PD-0 or modified form thereof shows at least one mutation distinct from the previously described CVB3 PD variants. It is believed - without being bound by the theory - that an exchange of at least one or more amino acid residues selected from the group consisting of amino acid residue K78, A80, A91, and I92 in the viral capsid protein 1 (VP1) and/or the amino acid residue M34 and Y237 in the viral capsid protein 3 (VP3) are responsible for the advantageous host range variations and the capacity of infecting HS expressing tumor cells. Accordingly, it has been shown that the inventive CVB3 PD strains (CVB3 PD-0) comprising one or several of these VP1 modifications and/or one or both of these VP3 modifications are capable of using HS and also subtypes of HS on target cell surface to mediate entry of the inventive CVB3 PD variant into target cells.

It had been previously shown that CVB3 Nancy induces severe inflammation of the pancreas and the heart in mice (Stein et al., 2015; Pinkert et al. 2009). In addition, CVBs are associated with the development of inflammatory and dilated cardiomyopathies in humans (Andreoletti et al., 2009; Andreoletti et al., 2007) and CVB3 Nancy has been described in connection with severe infections of children (Ronellenfitsch et al., 2014). Consequently, it seemed questionable to us to what extent CVB3 Nancy could actually be considered a safe OV.

It is therefore an object of the present invention to overcome at least partially the described disadvantages of the state of the art OVs in the treatment of cancer, especially with respect to efficiency and safety of used OVs. Moreover, it is the aim of the present invention to compare the oncolytic efficiency and safety of the CVB3 strains Nancy, H3, 31-1-93 and PD in treating human colorectal cancer as an exemplary candidate cancer type to be treated by CVB3 OVs.

This object has been solved by a method for treating colorectal cancer in a subject in need thereof, comprising administering to said subject a therapeutically effective amount of a modified Coxsackie B3 group (CVB3) virus according to claim 1.

An aspect concerns a novel method for treating cancer in a subject in need thereof, where the cancer can be characterized by comprising heparin sulfate expressing cells (either cancer cells or neighboring cells) and where the method comprises administering to said subject a therapeutically effective amount of a Coxsackie B3 group (CVB3) virus or a modified form thereof, which infects the cells of the cancer and/or in the cancer expressing a heparan sulfate (HS) receptor on their surface by binding of the virus binds to said HS receptor, entering and eventually lysing the infected cells, whereby at least some of the infected cells are cancer cells, which undergo viral lysis.

The Coxsackie B3 group virus is selected from the group of CVB3 Nancy, 31-1-93, H3 and PD, which have been modified by introducing at least one or several of the AA modifications in the VP1 and/or VP3 surface protein, which have been identified to be involved or responsible for the binding to the heparin sulfate receptor. The amino acid modifications may be due to a base pair exchange on the nucleic acid sequence and will lead to an alteration or exchange of at least one or more amino acid residues selected from the group consisting of amino acid residue K78, A80, A91, and I92 in the viral capsid protein 1 (VP1) and/or at least one or both of the amino acid residue M34 and Y237 in the viral capsid protein 3 (VP3) of CVB3. According to a preferred embodiment the Coxsackie B3 group virus is CVB3 PD-0 or a modified form thereof. CVB PD-0 comprises an exchange of at least one or more amino acid residues selected from the group consisting of amino acid residue K78, A80, A91, and I92 in the viral capsid protein 1 (VP1) and/or and at least one or both of the amino acid residue M34 and Y237 in the viral capsid protein 3 (VP3).

According to a preferred embodiment the modified CVB3 PD-0 form is rPD, rPD_{HiFi} or rPD-eGFP.

rPD: Without being bound by the theory, we hypothesized that a recombinant PD-0 cDNA clone contains less "mutation variants" after production *in vitro.* Typically, in cell culture amplified PD-0 will form "quasispecies" containing several virus "variants" of PD-0. This may increase the risk of undesirable side effects, e.g. infection of normal cells. Making use of a CVB3-PD-0 clone we expected to reduce this risk. Moreover, standardization of production of PD-0 can be better achieved by use of a PD-0 cDNA clone. For this approach CVB3 PD-0 full genome was cloned to obtain pure virus stocks of plasmid-based recombinant PD-0 (rPD) and to allow complete genome sequencing analysis.

rPD_{HiFi}:*in vivo.* Therefore, to increase the genome stability of rPD and to avoid unintended mutation, high fidelity replication variant of rPD (rPD_{HiFi}) was generated. As described by McDonald et al., 2016 phenylalanine which is the 364. amino acid of the 3D polymerase amino acid chain sequence was mutated to tryptophan by site directed mutagenesis using In-Fusion cloning kit (Takara Bio, Japan) with appropriate primers. rPD-eGFP : Reporter genes can be infused to the viral genome to detect or track virus replication. In addition, it also gives an overview about carriage capacity and the stability of the virus in case of exogenous gene insertion. Moreover, demonstrating its functionality when inserted into rPD it is the first step for the development oncolytic rPD which is armed with tumortoxic genes and may have higher oncolytic activity than rPD. Therefore, enhanced green fluorescent protein (eGFP) gene was inserted into the rPD. Firstly, Nrul restriction site incorporated modified 2A pro cleavage site (Lee et al., 2014) was inserted between P1 and P2 polyproteins of CVB3 genome. This plasmid was termed pPD_{2Apro}. EGFP was amplified from pMKS1-eGFP (Tong et al., 2011). pMKS1-eGFP contains full length genome of CVB3 strain H3 and encodes eGFP which was inserted to the downstream of start codon. pPD_{2Apro} was linearized with Nrul enzyme and ligated with amplified fragment encoding eGFP. This final plasmid was termed rPD-eGFP. rPD-eGFP was *in vitro* transcribed using T7 Transcription Kit (Roboklon Gmbh, Berlin, Germany) and RNA was isolated with RNeasy Mini Kit (Qiagen GmbH, Hilden, Germany).

A "subject in need" may be any mammal in need of treatment according to the invention. The subject may be a human or an individual of any species of social, economic or research importance including, but not limited to, mice, rats, dogs, cats, sheep, goats, cows, horses, pigs, non-human primates, and humans. In a preferred embodiment, the subject in need is a human.

In the present invention by the term "viral lysis" is meant, that a sufficient number of cells (at least one) lyse as a consequence of direct infection with at least one virus particle (direct viral lysis). Moreover, events leading to lysis of cancer cells without a former infection of this at least one cancer cell by an at least one virus particle, but caused or triggered by effects caused by the presence of virus-infected neighbouring cells - such cells possibly also being immune cells or other cell types being present in a tumor but not being cancer cells themselves (indirect viral lysis) are also included by that definition. The same is true also for e.g., a systemic anti-tumoral immune response caused by immunological processes induced by e.g. increased viral replication. A combination of direct lysis and indirect viral lysis events is particularly advantageous and included by this definition.

The "Coxsackie B3 group virus" according to the present invention may be any Coxsackie B3 group virus including known and classified Coxsackie B3 group viruses and yet to be classified Coxsackie B3 group viruses. The Coxsackie B3 group virus may be selected from the group consisting of both prototype and clinically isolated viruses. It may be naturally occurring or as claimed a "modified form thereof". The Coxsackie B3 group virus is "naturally-occurring" when it can be isolated from a source in nature and has not been intentionally modified in the laboratory - for instance the Coxsackie B3 group virus may be obtained from a human patient.

According to another embodiment of the invention the range of viral dose is between about 5×10⁵ to about 1×10⁸ plaque forming units (PFU), preferably between about 1×10⁶ to about 1×10⁷ PFU and most preferred between about 3×10⁶ to about 1×10⁷ PFU. It is also provided that the application of the viral dose is administered 2-, 3-, or 4-times depending of the size of the tumor, the patients response, the tumor regression rate and also the body weight of the patient. Typically, a dose of about 4.5 x 10⁶ PFU/kg for a 70-kg patient is to be considered.

According to the present invention, a Coxsackie B3 group virus or a modified form thereof or a nucleic acid molecule derived from a Coxsackie B3 group virus or a modified form thereof and combinations of the said may be administered either in a single dose, or in multiple doses. The multiple doses may be administered concurrently, or consecutively (e.g., over a period of days or weeks). Typically, in therapeutic applications the treatment would be for the duration of the disease condition, e.g. at least until the respective cancer is no longer detectable by conventional means. Typical treatment regimes are known in the state of the art.

The mode of administering of the inventive Coxsackie B group virus or a modified form thereof or a nucleic acid derived from the Coxsackie B3 group virus or a modified form thereof as well as combinations of the said to said subject in need thereof may be orally, intratumorally, intravenously, intraperitoneally and/or intramuscularly.

Surprisingly, the inventors have found that intratumoral injection of tumor bearing mice with any of the used virus strains led to significant inhibition of growth of the injected tumor. Even more surprisingly, a similar growth suppression was also observed for the distant, non-injected subcutaneous tumor. Whereas, these results demonstrate *per se* the efficacy of CVB3-mediated oncovirotherapy in a cancer type, which comprises cancer cells or other cells expressing and exposing heparin sulfate on the cell surface. According to one embodiment the invention showed to be particularly effective in treating colorectal cancer. Particularly advantageous seems to be the growth suppression of the untreated tumor, which reveals the very promising potential of the CVB3-strains according to the present invention for not only treating the primary tumor but also for treating metastatic or disseminated cancer.

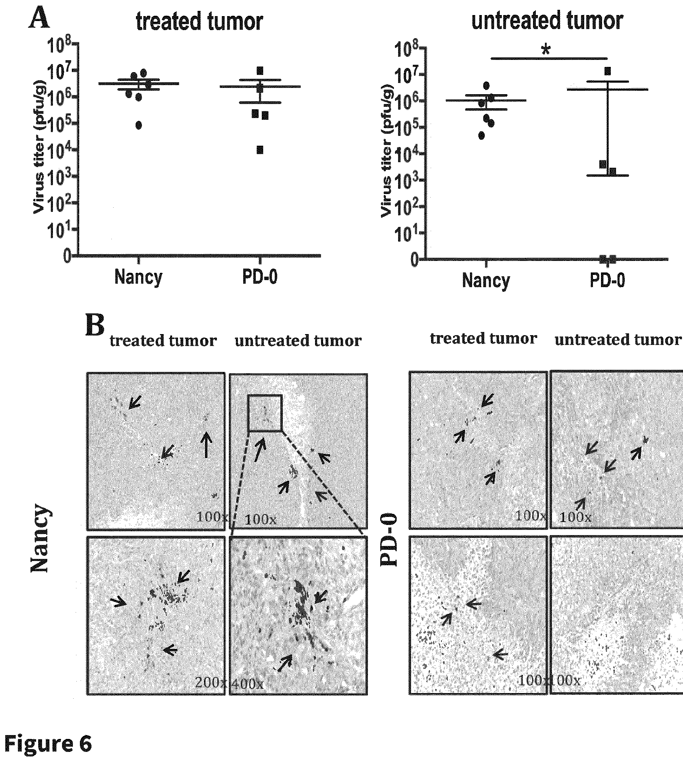

Surprisingly, the inventor could detect CVB3-PD-0 genomes in immune cells and their identified specific localization within the tumor mass or on the border between the tumor stroma and tumor cells (**Figure 6** B) suggests that virus-induced immune-mediated mechanisms may be involved in anti-tumor efficiency. Moreover, these findings may interestingly indicate - without being bound by that theory - that immune cells may be infected by the inventive virus strains and be or become carriers and a reservoir for hematogenous distribution of the therapeutic viruses within a tumor but also to distant tumors.

As mentioned above the way of administering the CVB3 PD virus of the invention is according to one embodiment an oral administration. This way of administering a therapeutic OV is particularly interesting for Coxackievirus in general but even more for the virus according the invention which has a strong host range tropism and preferably infects cells with an heparan sulfate receptor on the cell surface. Such heparan sulfate receptor expressing cells - preferably immune cells - can, thus, be used as carriers suitable to transporting the oncolytic CVB3 from a port of entry location e.g. in the Peyer's patches or the tonsils to the actual side of a tumor to be treated.

Most importantly, treatment with the inventive CVB3 PD virus or modified form thereof according to the present invention, -which are referred to in the following often with PD-0 - did not show any virus-induced side effects, especially no systemic infection. E.g., the inventors could show that virus could not be recovered from the organs of animals treated with the CVB3 PD virus or modified form. Therefore, herein disclosed findings reveal that the inventive PD-0 and variants thereof are an efficient and safe CVB3 strain for treatment of cancer cells according to the present invention. As CVB3 PD utilizes a heparin sulfate receptor, but Nancy and 31-1-93 need CAR and DAF to infect cells, the specific receptor tropism of the inventive CVB3 PD-0 is considered to be a important feature for the claimed method of treatment and its safety advantages, namely to prevent from any infection of normal tissues *in vivo.*

According to the present invention the CVB3-PD-0 virus or a modified form thereof or a nucleic acid molecule derived from said virus or a modified form thereof and combinations of the said may be administered in combination with one or more adjuvants or agents capable of modulating or suppressing an immune response in the subject to be treated. Thereby, preferably it may allow a more efficacious viral infection and/or viral lytic and/or therapeutic outcome. Typically, capable agents are well known by a person skilled in the art.

According to the present invention the CVB3 virus or a modified form thereof or a nucleic acid molecule derived from the CVB3 group virus or a modified form thereof and combinations of the said will be co-administered with a therapeutically effective amount of at least one secondary anticancer agent. Typically, capable anticancer agents are well known by a person skilled in the art. In an embodiment of the invention the at least one secondary anticancer agent is a chemotherapeutic agent. Typically, chemotherapeutic agents are well known by a person skilled in the art.

According to the present invention the CVB3 virus or a modified form thereof or a nucleic acid molecule derived from the CVB3 virus or a modified form thereof and combinations of the said will be used in combination with radiotherapy. Alternatively, radiotherapy can be combined with said at least one secondary anticancer agent.

Moreover, the present invention can be used in combination with therapeutic regimes and modalities well known by an artisan.

Interestingly, recent findings have shown that one or several of these four amino acid residues in VP1 are sufficient to allow infection and also induce "lytic" infections in both DAF- and CAR-negative cell lines - without being bound by this theory - suggesting that said at least one or several of these four modified residues in VP1 are the molecular basis of extended cell tropism of the inventive CVB3 PD variant and are involved in target cell receptor binding and entry into the target cell of CVB3 PD.

The cancer to be treated is selected from a cancer that has been identified as comprising cancer cells, which express heparan sulfate on the surface of the cancer cells. The exposure of heparan sulfates on the surface of cancer cells can be tested with commercially available heparan sulfate antibodies in e.g. flow cytometric methods, western bolts or Elisa assays. Heparan sulfate specific antibodies or Elisa kits are available from e.g. LSBio Inc., Seattle.

The cancer to be treated is selected from the group of colorectal carcinoma, esophageal cancer, breast cancer, lung cancer, pancreatic cancer, prostata cancer, gastric cancer and liver cancer. According to an embodiment the cancer is colorectal carcinoma. According to another embodiment of the invention the HS receptor is N- and/or 6-O-sulfated.

Interestingly, it was found that these specific heparan receptor modifications, i.e. N- or 6-O-sulfated heparan receptors at the surface of cancer cells can also mediate internalization of the inventive CVB3 PD variant.

It has been pre-described that there are three 6-O-sulfotransferases (6OST1-3) and seven 3-O-sulfotransferases triggering the 6-O- and 3-O-Sulfation of heparans in vertebrates. Accordingly, cancer comprising cancer cells with HS on the surface can also be detected or identified, e.g. after a cancer biopsy by the detection of the corresponding sulfotransferases or even by the detection of the RNA of such sulfotransferases in cancer cells.

Accordingly, the expression of heparan sulfate D-glucosaminyl 6-O-sulftransferase-2 (HS6ST2) enzyme, which is necessary for the synthesis of N- and/or 6-O-sulfated heparan receptors is one good marker for the suitability of cancer cells that can be treated effectively with the described method and the described CVB3 variants.

Additionally, HS6ST2 mRNA expression was hardly or not at all found being expressed in respective non-cancerous cell tissues. This may be a promising finding as this would argue for an increased safety of the use of CVB3 viruses or modified forms thereof according to the present invention. This, because of the use of N- and 6-sulfated heparan receptors at a cell surface for infection of that cell by a virus according to the present invention, wherein the expression of N- and 6-O-sulfated heparan surface receptors is (essentially) restricted - without being bound by that theory - to the surface of cancer cells. This in turn hinders uncontrolled infection of non-cancerous tissue. lacking the expression of said heparan receptors, thereby increasing the safety of said virus and said nucleic acid, derived from said virus for medical usage.

Also, for the specific heparin sulfate receptor, namely N- and 6-O-sulfated heparan, which can be found at a cell surface of cancer cells suitable for treatment according the present invention anti N- and 6-O-sulfated heparan receptor antibodies are well known and commercially available e.g. by LSBIO Inc., Seattle. Western blots or ELISAs using these antibodies may be a proper mean to prove expression of N- and 6-O-sulfated heparan receptor expression in cancer cells relative to control samples, i.e. respective non-cancerous tissue samples, e.g. colorectal cancer cells relative to non-cancerous colorectal tissue sample. Also, antibodies detecting the presence of anti-Heparan Sulfate 6-O-Sulfotransferase 3 (HS6ST3), the enzyme, which is necessary for the synthesis of N- and 6-O-sulfated heparan receptors may be used as expression indicator for 6-sulfated heparan receptors.

This is especially of interest if cells of a target cancer will substantially lack expression of the CAR and/or the DAF on their surface. The more as certain CVB3 variants, which are dependent on CAR and/or DAF to enter the cells, cannot enter cells if these cells essentially lacking expression of one or both of said receptors. However - without being bound by that theory - the inventive CVB3 PD variants and modified forms thereof, which only need expression of certain heparan sulfated receptors for infection would be capable to infect cancerous cells expressing these heparan sulfated receptors.

Therefore, according to an embodiment of the invention the treatment is particularly suitable for cells of a cancer, wherein the cancer cells substantially lacking expression and/or exposure of the Coxsackievirus and adenovirus receptor (CAR) or the decay-accelerating factor (DAF) on their surface.

The term "substantially lacking expression" refers to a protein expression on cell surface in which all or substantially all cells, e.g. more than 70%, more than 75%, more than 80%, more than 85%, more than 90%, more than 95%, more than 98%, more than 99%, or 100% of a cancer of a certain tissue origin do not express said respective proteins on their surface compared to the surface expression level of the respective normal (non-cancerous) tissue of origin as measured by flow cytometry using commercially available antibodies, precisely commercially available anti-DAF- or anti-CAR-antibodies, e.g. those as used herein.

According to another embodiment of the invention the cells of the cancer show some expression of the CAR or the DAF on their surface.

In the context of the present invention under the term "therapeutically effective amount" is meant, that a Coxsackie B3 group virus or a modified form thereof respectively a nucleic acid derived from a Coxsackie B3 group virus or a modified form thereof or combinations of said virus and said nucleic acid is administered in an amount that is sufficient to treat the respective cancer.

A cancer according to the inventions of the present invention is "treated", if administration of the said virus or the said nucleic acid to cells of the respective cancer effects viral lysis of at least some of the cancer cells, resulting in at least a reduction of tumor burden, tumor growth or tumor cell viability and/or preferably in an increase of the life span of a treated subject in need thereof. The effective amount will be determined on an individual basis and may be based at least in part, on consideration of the CVB3 virus or modified form thereof, the body weight, age, gender of the subject to be treated (subject in need thereof) and the type, extent and other characteristics of the respective cancer to be treated.

The CVB3 virus or modified form thereof or nucleic acid molecule derived from the Coxsackie B3 group virus or a modified form thereof may be administered to a respective cancer in the individual subject in need thereof. A combination of different serotypes and/or different strains and/or different species and/or different genera of CVB3 virus, such as CVB3 virus from different species of animal, may be used. If desired, the Coxsackie B group virus can be chemically or biochemically pretreated, e.g., by treatment with a protease, such as chymotrypsin or trypsin prior to administration to the neoplasm. Such pretreatment will lead to removal of the outer coat of the virus and may thus result in improved infectivity of the virus. Combinations of at least two different CVB3 viruses and/or modified forms thereof as well as combinations of at least two different nucleic acids derived from the CVB3 virus and/or modified forms thereof may be administered. Also, combinations of the said at least two different CVB3 viruses and/or modified forms thereof together with at least one nucleic acid derived from the CVB3 virus and modified form thereof may be administered. And, combinations of the said at least two different nucleic acids derived from the CVB3 virus and/or modified form thereof together with at least one CVB3 virus and/or modified forms thereof may be administered. The CVB3 virus or modified form thereof may be administered or applied in combination with other additional viruses. E.g., the applied CVB3 virus or modified form thereof will be administered with at least one strain or serotype or species or genera of Coxsackie B virus or modified forms thereof, which have either the same receptor requirements for cell infection as the CVB3 virus or modified form thereof of the invention, i.e. the HS receptor tropism or different receptor requirements.

According to an aspect the CVB3 virus genome sequence comprises at least one or several modified nucleic acid molecule.

In the sense of the present invention a "nucleic acid molecule derived from a Coxsackie B3 group (CVB3) virus or a modified form thereof" may be understood as to include any viral nucleic acid, e.g. viral vectors, the complete viral RNA genome or a sufficient part thereof including complementary DNA (cDNA) versions of the said to permit generation of a lytic response in virus-infected cancer cells or generation of new viral particles, which then will cause a lytic response in the cell and be release ready for the infection of further - sometimes - neighboring cells.

The viral nucleic acid sequence can be used as vector or can incorporate additional transgenic or therapeutic nucleic acid molecules, which are selected from the group of therapeutic transgenes or small RNAs. Therapeutic transgenes, may comprise a RNA transcript of therapeutically interesting cDNAs or other small RNAs, e.g. microRNA, siRNAs (e.g. microRNA-target sites (miR-TS)), which are well known by an artisan.

As a result of the incorporation of transgenic or therapeutic nucleic acid molecules into the genome or nucleic acid sequence of the CVB3 virus an aspect provides recombinant Coxsackie B3 group (rCVB3) viruses.

According to another embodiment the CVB3 PD or a modified form thereof comprise nucleic acid sequence modifications giving rise to at least one or several modified amino acid residues selected from the group of amino acid residues K78, A80, A91, and I92 in the viral capsid protein 1 (VP1) and/or at least one or both of the amino acid residue M34 and Y237 in the viral capsid protein 3 (VP3).

According to one interesting embodiment of the invention the treatment even works, where cancer cells in the cancer to be treated do substantially lack a HS expression or exposure on the cell surface and at the same time in this tumor are neighboring cells (immune cells or other cell types, which do express HS on their surface. In this case the CVB3 virus of the invention will be infecting the neighboring cells (so called bystander cells) and will in a first step lyse such cells, subsequently and due to the amount of virus present in the cancer or at the location of the tumor, also the cancer cells will become infected and lysed. Accordingly, the present invention also provides in one embodiment a therapy for the treatment of a tumor, which does not comprise cancer cells expressing HS on the surface, but which (as shown in a biopsy) is infiltrated by other cells which do express HS on their surface and which therefore provide after the infection with the virus of the invention intratumoral virus production centers. In this case it is helpful but not necessary if the cancer cells would be capable of expressing and presenting on their cell surface the Coxsackievirus and adenovirus receptor (CAR) and/or the decay-accelerating factor (DAF) on their surface. Accordingly, in one embodiment of the invention the cells of the cancer show expression of the CAR or the DAF on their surface.

Beside the above-described therapeutic approach, an aspect also provides the treatment of a target cancer, which is substantially lacking expression of the CAR and/or the DAF on their surface. This is particularly the case, because CVB3 PD-0, which only needs expression of heparan sulfated receptors for infection - without being bound by that theory - is capable to infect such cancer cells expressing these heparan sulfated receptors.

This is particularly advantageous as other CVB variants, which are dependent on CAR and/or DAF to enter the cells, cannot enter such cancer cells lacking expression of one or both of said receptors.

According to one further embodiment of the method of the present invention the HS receptor is a N- and/or a 6-O-sulfated heparan.

Suitable pharmaceutically acceptable excipient, diluent and carriers are well known by an artisan. In brief, examples of pharmaceutically acceptable carriers or diluents are demineralised or distilled water; saline solution; vegetable based oils such as peanut oil, safflower oil, olive oil, cottonseed oil, maize oil, sesame oils such as peanut oil, safflower oil, olive oil, cottonseed oil, maize oil, sesame oil, arachis oil or coconut oil; silicone oils, including polysiloxanes, such as methyl polysiloxane, phenyl polysiloxane and methylphenyl polysolpoxane; volatile silicones; mineral oils such as liquid paraffin, soft paraffin or squalane; cellulose derivatives such as methyl cellulose, ethyl cellulose, carboxymethylcellulose, sodium carboxymethylcellulose hydroxypropylmethylcellulose; lower alkanols, for example ethanol or iso-propanol; lower aralkanols; lower polyalkylene glycols or lower alkylene glycols, for example polyethylene glycol, polypropylene glycol, ethylene glycol, propylene glycol, 1,3butylene glycol or glycerin; fatty acid esters such as isopropyl palmitate, isopropyl myristate or ethyl oleate; polyvinylpyrolidone; agar; gum tragacanth or gum acacia, and petroleum jelly. Typically, the carrier or carriers will form from 10% to 99.9% by weight of the pharmacological compositions.

Methods for preparing parenterally administrable compositions are apparent to those skilled in the art, and are described in more detail in, for example, Remington's Pharmaceutical Science, 15th ed., Mack Publishing Company, Easton, Pa., hereby incorporated by reference.

Some examples of suitable carriers, diluents, excipients and adjuvants for oral use include peanut oil, liquid paraffin, sodium carboxymethylcellulose, methylcellulose, sodium alginate, gum acacia, gum tragacanth, dextrose, sucrose, sorbitol, mannitol, gelatine and lecithin. In addition these oral formulations may contain suitable flavouring and colouring agents. When used in capsule form the capsules may be coated with compounds such as glyceryl monostearate or glyceryl distearate, which delay disintegration.

The pharmacological composition may incorporate any suitable surfactant such as an anionic, cationic or non-ionic surfactant such as sorbitan esters or polyoxyethylene derivatives thereof. Suspending agents such as natural gums, cellulose derivatives or inorganic materials such as silicaceous silicas, and other ingredients such as lanolin, may also be included.

### Short description of the Figures

**Figure 1****.** Model for the successful generation of the inventive CVB3 PD by serial passaging.
**Figure 2**. (A) nucleic acid sequence of the inventive CVB3 PD-0, including several differences in nucleotides towards the nucleotide sequence of state of the art CVB3-PD (GenBank ID: AF231765.1; https://www.ncbi.nlm.nih.gov/nuccore/AF231765) are indicated by small letters, bold and underlines; to indicate different sequence parts or regions within the continuous sequence, the continuous sequence is here presented in "sections". A section is introduced by the "index number of the section/sequence part" and illustrates a given sequence, which is surrounded by an empty line (above and) underneath, legend: First sequence part: 5UTR, Second sequence part: VP4, Third sequence part: VP2, Fourth sequence part: VP3, Fifth sequence part: VP1; In comparison to the CVB3-PD sequence in The Genbank (accession number: AF231765), the inventive CVB3 variant, PD-0 contains following nucleotide differences: C610T, A1801G, C1839T, C2248A, A2965G. The first letter indicates the nucleotide according to GenBank sequence, last letter indicates the nucleotide according to our sequence, number indicates the position of nucleotide.
   (B) amino acid sequence of the inventive CVB3 PD-0, including several differences in amino acid residues towards the protein sequence of state of the art CVB3-PD (GenBank ID: AF231765.1; https://www.ncbi.nlm.nih.gov/nuccore/AF231765) are indicated by bold and underlines; to indicate different sequence parts or regions within the continuous sequence, the continuous sequence is here presented in "sections". A section is introduced by the "index number of the section/sequence part" and illustrates a given sequence, which is surrounded by an empty line (above and) underneath, legend: First sequence part: VP4, Second sequence part: VP2, Third sequence part: VP3, Fourth sequence part: VP1;

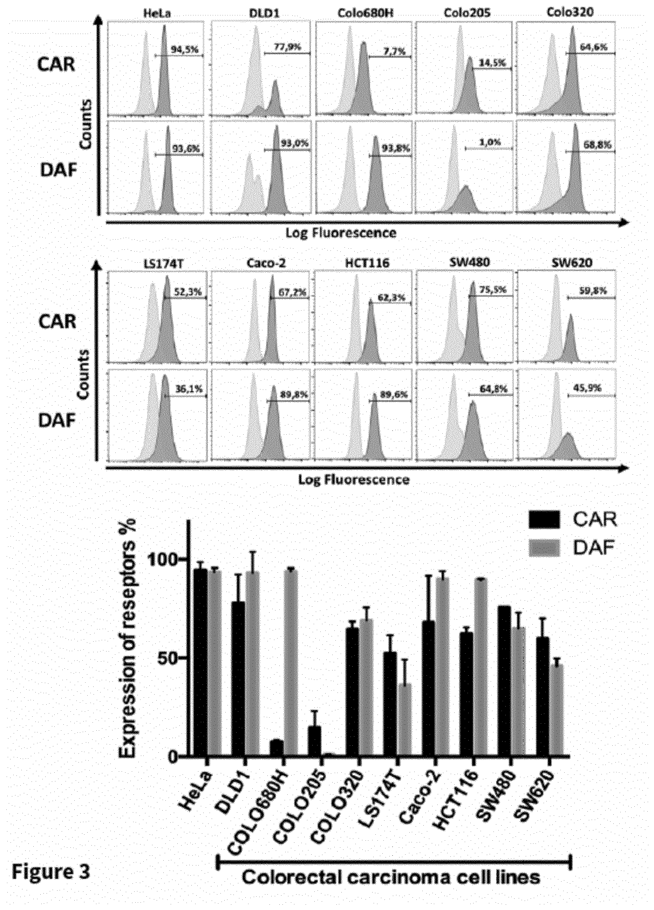

**Figure 3****.** Expression level of surface CAR and DAF in colorectal carcinoma cell lines and HeLa cell line. Upper panel: Cell surface expression of CAR and DAF (dark gray graphs) was measured by flow cytometry using anti-CAR (RMCB) and anti-DAF (CD55) antibodies, respectively. As negative control (light gray graph), cells were stained only with the secondary antibody (Alexa Fluor^{®} 488). HeLa cells were used as positive control due to high level expression of CAR and DAF. The black highlighted area represents the set gate. The percentages indicate the proportion of CAR or DAF positive cells. Lower panel: Diagram of cell surface expression of CAR and DAF in colorectal carcinoma cells. Columns shown means ± SEM of three independent experiments.

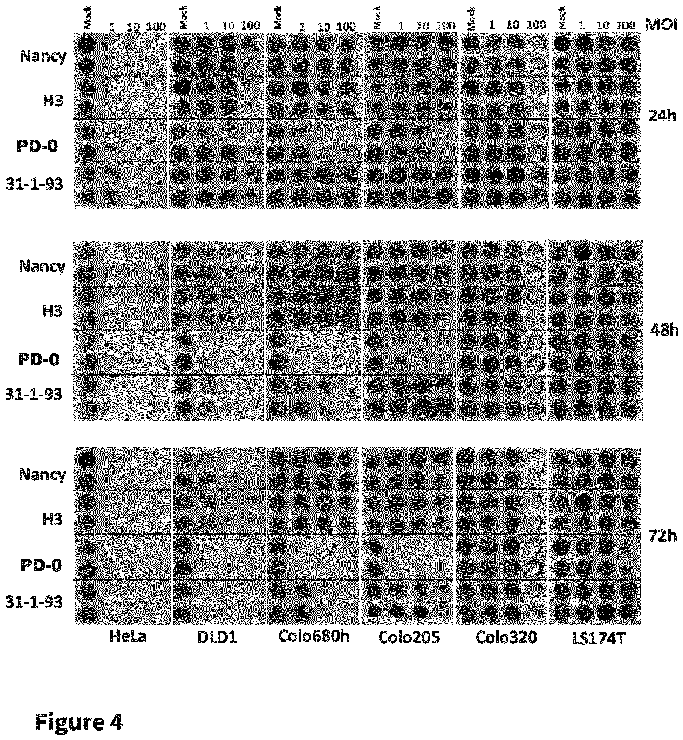

**Figure 4****.** CVB3 strains have different oncolytic efficacy against colorectal carcinoma cells *in vitro.* The cell killing assay was performed with CVB3 Nancy, H3, PD-0 and 31-1-93 strains. HeLa cells and DLD1, Colo680H, Colo205, Colo320, and LS174T colorectal carcinoma cells were infected with either 1, 10 or 100 MOI of virus. Cell viability was determined 24 h, 48 h and 72 h post infection by crystal violet staining.

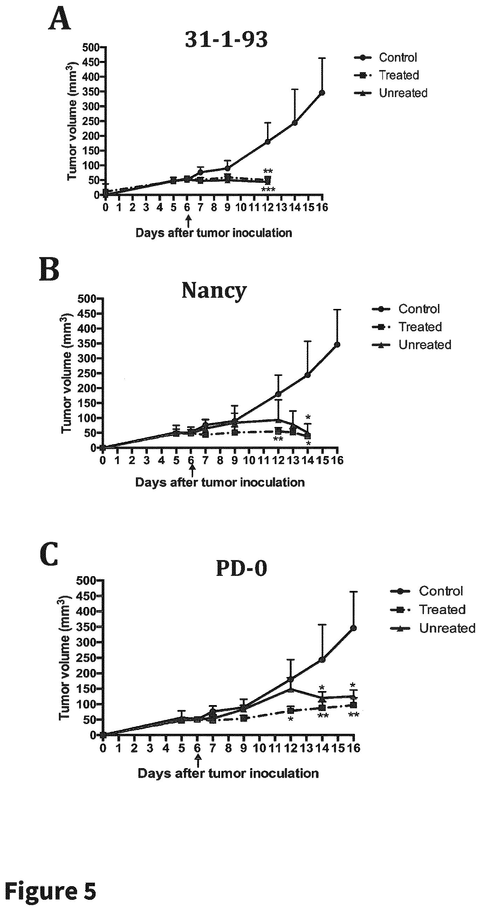

**Figure 5****.** *In vivo* oncolytic efficiency of inventive CVB3 strains in DLD1 xenograft mouse model. DLD1 cells were subcutaneously inoculated into the right and left flank of BALB/c nude mice. One of the inoculated tumor was intratumorally injected with single dose (3 x 10⁶ PFU) of the CVB3 strains (A) 31-1-93 (n = 6), (B) Nancy (n = 6) and (C) PD-0 (n = 5), respectively, at 6 days after tumor inoculation. Control group (n=4) was not treated. Tumor volumes are shown as means ± SEM. CVB3 31-1-93 infected mice died 6 days after viral injection. CVB3 Nancy infected mice were sacrificed at day 8 after viral injection, at which time they were moribund, CVB3 PD-0 and un-injected control mice were sacrificed at day 10 after viral injection. *,P<0.05; **, P<0.01; ***,P<0.001 compared to the control.
**Figure 6****.** Infectivity and replication activity of CVB3 Nancy and PD-0 strain in harvested tumors A) Left: virus titers in CVB3 Nancy and PD injected tumors. Right: virus titers in the un-injected tumor, * P<0.05; n.s. not significant; B) Presence and localization of CVB3-Nancy and PD-0 RNA treated and untreated tumors were determined by *in situ* hybridization using DIG-labeled RNA hybridization probes. Magnification, 100-fold.

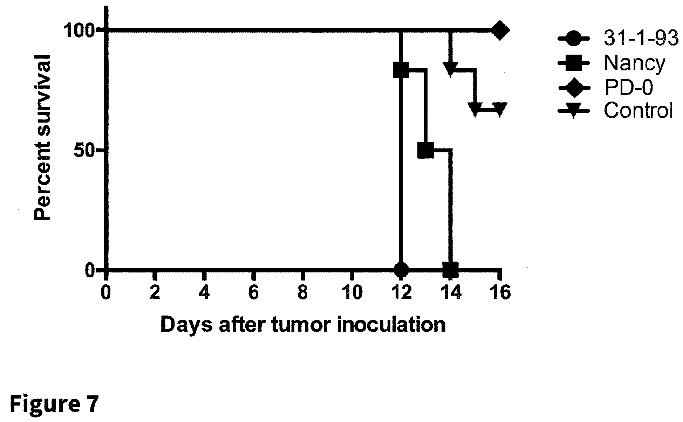

**Figure 7****.** Kaplan-Meier survival curves for the mice. Animals died or were sacrificed as described under Figure 5. The control group mice were sacrificed when the tumor burden reached an upper limit of 500 mm³ for reasons of animal welfare. P = 0.0006.

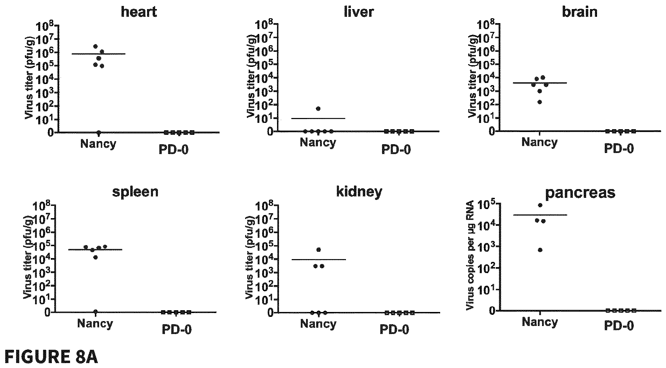

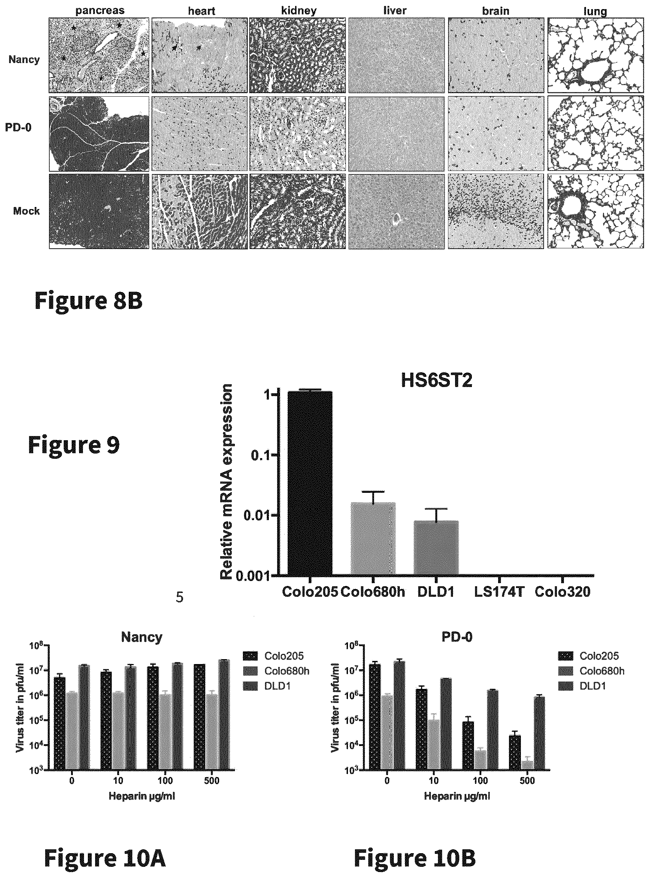

**Figure 8****.** Viral load and pathological alterations of CVB3 Nancy and PD-0 in organs of tumor bearing mice. Organs of the animals were harvested at the time points when animals were sacrificed as described under Figure 5. A) Amount of replicating virus. Plaque assays for the detection of replicating virus was performed for the heart, liver, spleen, brain and kidney, whereas the amount of virus in the pancreas was determined by quantitative RT-PCR. Note: Because of almost complete tissue destruction, pancreas could be harvested from only four of six animals of the CVB3 Nancy treated group for viral genome detection. B) Histological examination of mouse tissues. Organ sections were stained with hematoxylin and eosin (magnification of images 200-fold). All Nancy infected animals showed complete destruction of the exocrine pancreas (stars represent areas of tissue destruction). Inflammation within the myocardium (arrows) was detected in all Nancy infected animals. All PD-0 infected animals were free of pathological alterations in all investigated organs. Mock represent untreated tumor bearing animals. No alteration of tissues was seen in these animals. Shown are representatives of each group.
**Figure 9****.** Expression of HS6ST2 mRNA in Colo205, Colo680h, DLD1, LS174T and Colo320 human colorectal cancer cell lines. Relative HS6ST2 mRNA expression levels were determined using quantitative RT-PCR. 18S RNA was used as internal control. HS6ST2 mRNA levels were normalized against 18S mRNA expression levels.
**Figure 10****.** Heparan sulfate mediates infection of PD-0. (A) Effect of heparin on infection of colorectal cancer cell lines by CVB3 (strain Nancy which uses the coxsackievirus and adenovirus receptor (CAR) to infect cells) and (B) PD-0. Nancy and PD-0 (MOI of 3) were incubated with heparin for 1 h and added to the cells. Virus infection was analyzed by plaque assay after 48h later. Heparin did not inhibit the infection of cell lines with CVB3 Nancy, whereas PD-0 infection was strongly inhibited in dose-dependent manner.

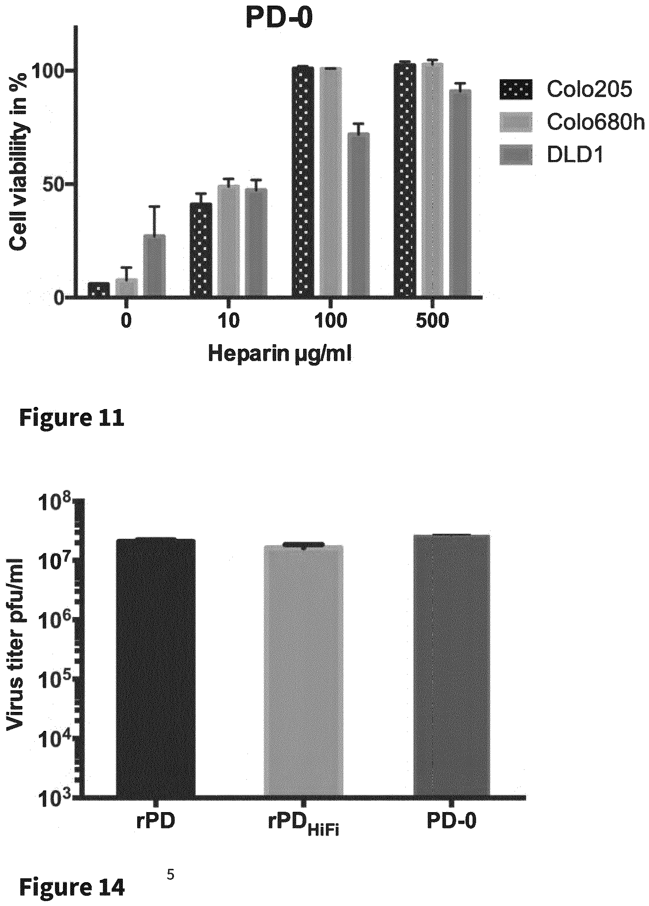

**Figure 11****.** Heparin inhibits PD-0 induced cytotoxicity in human colorectal cancer cell lines. Cell viability was measured 48 h after infection using XTT assay. Strongest inhibition was observed Colo205 and Colo680h. Inhibition in DLD1 cell line was lower at high heparin concentrations (100 µg/ml and 500 µg/ml) probably due to high level of CAR on cell surface, as PD, as PD-0 can also use CAR for infection.

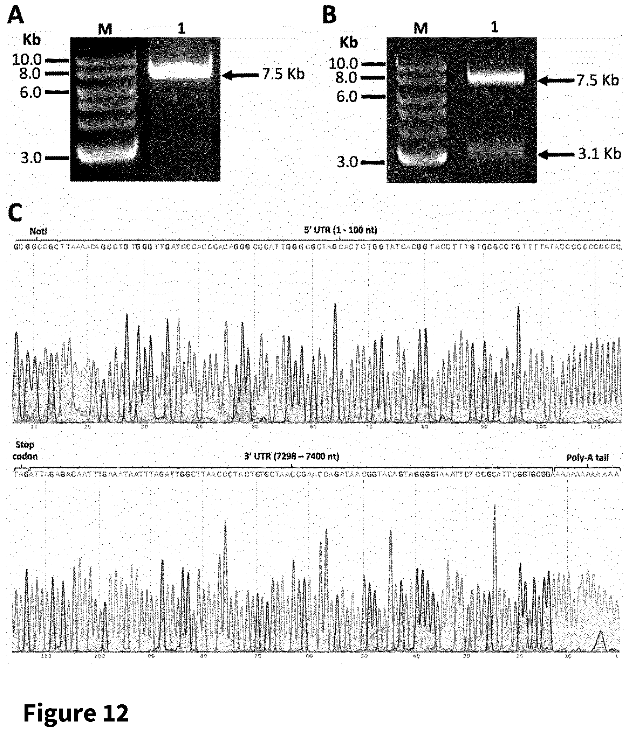

**Figure 12****.** Construction of cDNA clone of infectious CVB3-PD-0. **A)** Viral RNA was reverse transcribed, followed by PCR reaction for full genome amplification. 7,5 kb long final product was confirmed on agarose gel. Lane M; 1-log DNA ladder, Lane 1; PCR product. **B)** PCR fragment was extracted from the gel and inserted into the pJET1.2/blunt cloning vector. This plasmid was termed rPD. Plasmid DNA was recovered from bacteria culture and digested with Notl and Clal restriction enzymes to confirm product. Lane M; 1-log DNA ladder, Lane 1 upper band; full length PCR fragment derived from PD-0, lower band; vector backbone. **C)** Chromatograms of standard Sanger sequencing of 5'UTR and 3'UTR of CVB3-PD-0 insert. Sequencing was carried out with pJET1.2 forward and reverse primers spanning insertion site of plasmid. Upper sequence chromatogram represents the first 100 nucleotide of 5' UTR region of PD-0 genome. Bottom chromatogram represents 3'UTR region of PD-0 genome together with poly-A tail.

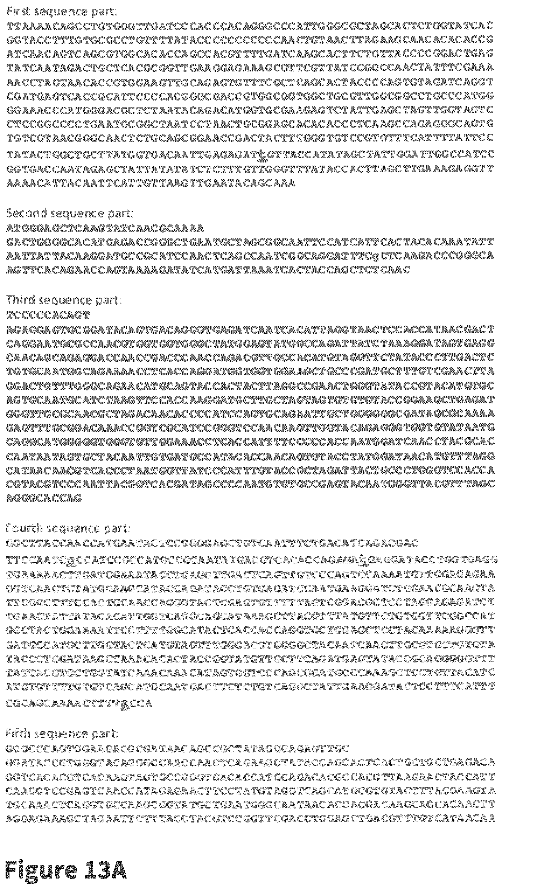

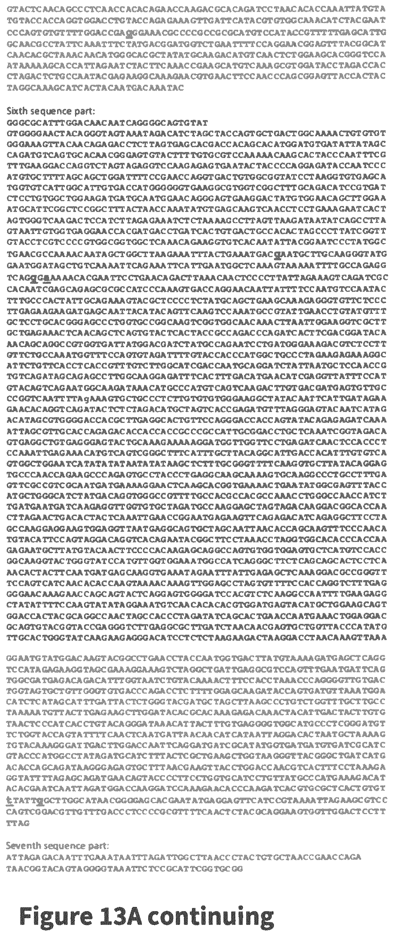

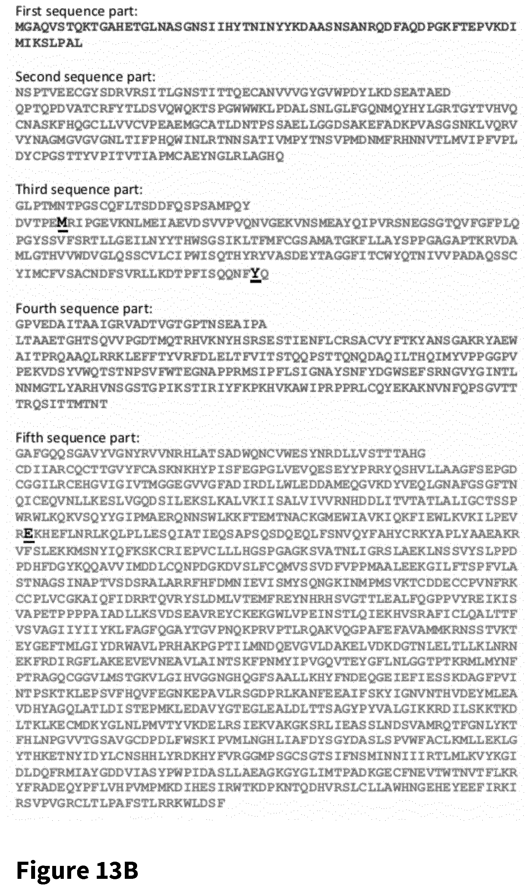

**Figure 13****.** (A) nucleic acid sequence of the inventive full length cDNA CVB3 PD-0 containing plasmid-based recombinant PD (rPD), including several differences in nucleotides towards the nucleotide sequence of state of the art CVB3-PD (GenBank ID: AF231765.1; https://www.ncbi.nlm.nih.gov/nuccore/AF231765) are indicated by small letters, bold and underlines; to indicate different sequence parts or regions within the continuous sequence, the continuous sequence is here presented in "sections". A section is introduced by the "index number of the section/sequence part" and illustrates a given sequence, which is surrounded by an empty line (above and) underneath, legend: First sequence part: 5UTR, Second sequence part: VP4, Third sequence part: VP2, Fourth sequence part: VP3, Fifth sequence part: VP1; In comparison to the CVB3-PD sequence in The Genbank (accession number: AF231765), the inventive full length cDNA CVB3 variant, PD-0 contains following nucleotide differences: C610T, C881T, A1801G, C1839T, C2248A, A2965G, A4078G, A4165G, G4167A, A4878G, C7169T and A7174G. The first letter indicates the nucleotide according to GenBank sequence, last letter indicates the nucleotide according to our sequence, number indicates the position of nucleotide.
   (B) amino acid sequence of the inventive full length cDNA CVB3 PD-0 containing plasmid-based recombinant PD (rPD), including several differences in amino acid residues towards the protein sequence of state of the art CVB3-PD (GenBank ID: AF231765.1; https://www.ncbi.nlm.nih.gov/nuccore/AF231765) are indicated by bold and underlines; to indicate different sequence parts or regions within the continuous sequence, the continuous sequence is here presented in "sections". A section is introduced by the "index number of the section/sequence part" and illustrates a given sequence, which is surrounded by an empty line (above and) underneath, legend: First sequence part: VP4, Second sequence part: VP2, Third sequence part: VP3, Fourth sequence part: VP1.
**Figure 14****.** Viral titer of plasmid based PD variants and PD-0. Plaque assay was performed in HeLa cells for titration of propagated rPD and rPD_{HiFi} and compared with PD-0 stock titer. Both plasmid based PD variants reached to the same titer of wtPD.

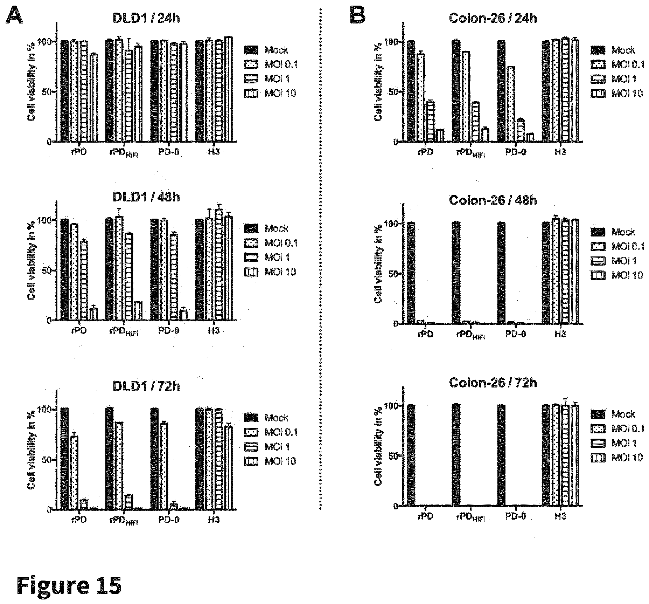

**Figure 15****.** Cell viability assay for DLD1 and Colon-26. A) DLD1 human colorectal carcinoma cells or B) Colon-26 murine colorectal carcinoma cells were infected with rPD, rPD_{HiFi}, PD-0 or H3 in different MOIs (0.1, 1 and 10). Cell viability was assessed 24h, 48h and 72h post virus infection. Results confirmed that oncolytic activity of plasmid based PD variants retained the oncolytic activity.

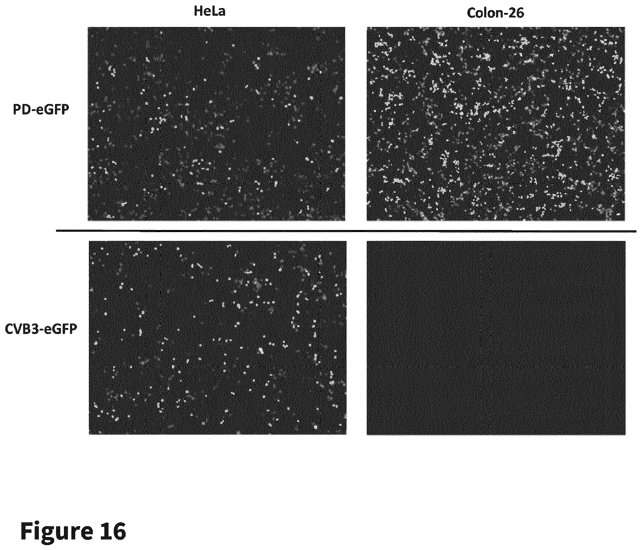

**Figure 16****.** Expression of eGFP in HeLa and Colon-26 cells. Upper row, HeLa and Colon-26 cells were infected with rPD-eGFP (MOI of 0.5) and eGFP expression was monitored 24h post infection by florescent microscope. Lower row, HeLa and Colon-26 cells were infected with CVB3-eGFP (MOI of 0.5) and eGFP expression was monitored 24h post infection by florescent microscope. CVB3-eGFP is derived from CVB3 H3 strain, therefore couldn't infect the Colon-26 murine colorectal cell lines as shown in Figure 15.

### EXAMPLES

In the following the invention will be described in more detail by reference to specific examples, which should not be construed as in any way limiting the scope of the present invention.

### Cell Lines

HeLa cells were cultured in Dulbecco's modified Eagle's medium (DMEM) (Gibco BRL, Karlsruhe, Germany) supplemented with 5% fetal calf serum (FCS) and 1% penicillin-streptomycin. Colorectal carcinoma cell lines (DLD1, Colo205, Colo680h, Colo320) were grown in RPMI 1640 supplemented with 10% FCS, 1% penicillin-streptomycin, 1% L-Glutamine and 1mM Na-Pyruvate (Invitrogen, Karlsruhe, Germany). LS174T cells were maintained in EMEM (Lonza, Basel, CH) and supplemented with 10% FCS, 1% penicillin-streptomycin, 1% L-Glutamine and 1mM Na-Pyruvate (Invitrogen, Karlsruhe, Germany).

### Viruses

The inventive CVB3 strain PD was derived by serial passages of the CVB3 strain P on HuFi cells (26). The cardiotropic CVB3 strain 31-1-93 was isolated from heart tissue after four heart passages of PD in outbred NMRI mice. CVB3 PD and 31-1-93 were propagated in CAR/DAF-negative CHO-K1 and in HeLa cells, respectively, before use. CVB3 strain Nancy (ATCC VR30) was propagated in HeLa cells. CVB3 H3 was generated by transfection of the cDNAcontaining plasmid pBK-CMV-H3 into HEK293T cells using Polyethylenimine Max (Polyciences, Warrington, PA, USA). Completely lysed cells were harvested 48 h post transfection and the virus was stored in aliquots after three freeze/thaw cycles and removal of the cell debris by centrifugation.

### Viral plaque assay

Viral plaque assays were carried out as previously described (Fechner et al., 2008). Briefly, HeLa cells were cultured in 24-well cell culture plates as confluent monolayers. After 24 h, medium was removed and cells were overlaid with serial ten-fold dilutions of supernatant harvested from virus-infected cell lines or from homogenized mouse organs after 3 freeze/thaw cycles. Cells were then incubated at 37° C for 30 min and, after removal of the supernatant, overlaid with agar containing Eagle's minimal essential medium (MEM). Three days later, the cells were stained with 0.025% neutral red in phosphate-buffered saline (PBS). Virus titers were determined by plaque counting 3 h after staining.

### Cell Killing Assay

Colorectal carcinoma cells were seeded in 96-well plates and, on the following day, when cells reached complete confluence, the medium was carefully removed and virus solution (100µl) was added at multiplicity of infection (MOI) of 1, 10 and 100. After 30 min incubation at 37° C and 5% CO2, virus-containing medium was removed and fresh medium was added and cells incubated for different periods. To fix the cells, the medium was removed, the cells were washed with PBS and 10% trichloroacetic acid (TCA) (Carl Roth, Karlsruhe, Germany) was added. Following an incubation of 10 min, TCA was removed and 30 µl crystal violet solution (Carl Roth, Karlsruhe, Germany) was added. After 5 min incubation, wells were washed with PBS several times, the plate was allowed to dry overnight and photographed.

### CVB3 RNA quantification

Colorectal carcinoma cells were seeded in 96 well plates and infected with CVB3 strains. Twenty-four hours later plates were subjected to three freeze/thaw cycles and the collected supernatant centrifuged for 20 min at 2,000 x g and 4° C to remove cellular debris. Viral RNA was isolated from the supernatant with High Pure viral nucleic acid kit (Roche, Mannheim, Germany) according to the manufacturer's instructions, followed by DNase I digestion (Peqlab, Erlangen, Germany). The viral RNA was reverse transcribed using the High-Capacity cDNA reverse transcription kit (Applied Biosystems Inc., Foster City, CA, USA). For quantification of viral RNA, real-time PCR was performed using CFX96 Real-Time System combined with a C1000 Thermal Cycler (Bio-Rad), using the forward primer, 5'-CCCTGAATGCGGCTAATCC and the reverse primer 5'-ATTGTCACCATAAGCAGCCA in Sso Fast^{™} EvaGreen Supermix (Bio-Rad). Cycle times were as follows: one cycle at 50 °C for 2 min followed by 94 °C for 10 min, 40 cycles at 94 °C for 15s, and 60 °C for 60s. A standard curve was used to calculate the number of CVB3 genome copies. The standard curve was prepared using 10-fold serial dilutions of 146 bp PCR fragment of CVB3 plasmid, which was amplified with the forward and reverse primers mentioned above.

### Flow Cytometric Analysis of CAR and DAF Expression

Colorectal carcinoma cells were washed with PBS and detached from cell culture plates using PBS-2mM EDTA solution. After washing with PBS the cells were stained on ice with monoclonal mouse anti-CAR/IgG1 (clone RmcB) antibody (Merck KGaA, Darmstadt, Germany) or with monoclonal mouse anti-DAF/CD55 (Merck) for 1 h at a dilution of 1:200. Cells were washed again with PBS and resuspended in Alexa Fluor 488-conjugated donkey anti-mouse IgG antibody (Life Technologies GmbH, Darmstadt, Germany), which was diluted 1:400 in PBS, and incubated for 45 min. After a further wash step with PBS, the cells were resuspended in PBS + 1 % formaldehyde and analyzed by flow cytometry using a MACSQuant^{®} Flow Cytometers (Miltenyi Biotec GmbH, Bergisch Gladbach, Germany) and FlowJo, Data Analysis Software (Tree Star, San Carlos, CA, USA). The mean fluorescence intensity was calculated by determining the geometric mean of CAR or DAF-expressing cells minus the geometric mean of a negative control sample.

### In situ Hybridization

Probes for the detection of CVB3 RNA by in situ hybridization were generated using the Dig RNA labeling Kit (Roche, Mannheim, Germany) using the pCVB3-R1 plasmid, which was linearized with *Sma*I as previously described (28). Pretreatment, hybridization and washing steps were done as described previously (29). Detection of the DIG-labeled RNA-probe was performed with a horseradish peroxidase-conjugated anti-Digoxigenin antibody (Roche, 1:100) and HistoGreen (Linaris GmbH, Dossenheim, Germany) as substrate. Sections were counterstained with hematoxilin and mounted with Pertex mounting media (Medite, Burgdorf, Germany).

### In vivo experiments

Animal experiments were performed in accordance with the principles of laboratory animal care and all German laws regarding animal protection. DLD1 cells (5 x 10⁶ cells) were inoculated subcutaneously into the right and left flanks of 6 weeks old BALB/c nude mice. Tumor burdens were measured daily by hand caliper and CVB3 strains was injected intratumorally at a dose of 3 x 10⁶ plaque forming units (PFU) per animal into one of the tumors when the tumor size reached 0.4 - 0.5 cm.

### Histopathological Analysis

The mouse tissues and explanted human tumors were fixed in 4% paraformaldehyde, embedded in paraffin, and 5 µm thick tissue sections were cut and stained with hematoxylin and eosin (H&E) to visualize and quantify cell destruction and inflammation.

### Statistical Analysis

Statistical analysis performed with GraphPad Prism 5.03 (GraphPad Software, Inc., La Jolla, CA, USA). Results are shown as mean ± SEM for each group. Statistical significance was determined by use of the two-tailed unpaired Student t test for cell culture investigations and by use of the one-tailed Mann-Whitney U-test for *in vivo* investigations. Differences were considered significant at P<0.05. Survival curves were plotted according to Kaplan-Meier method (log-rank-test).

### Example 1: Functionality and construction of CVB3 PD

The Applicant received the starting virus PD from Michaela Schmidtke, (Institute of Virology and Antiviral Therapy, University of Jena), which sequence has been published (GenBank ID: AF231765.1; https://www.ncbi.nlm.nih.gov/nuccore/AF231765). The virus replicates in CAR / DAF-negative CHO cells and can be propagated in them. The virus was partially sequenced after multiplication.

A comparison of the PD variant used herein and the state of the art sequence of PD was performed. There, it was shown that the inventive PD variant used herein has in the VP3 protein a mutation towards the PD variant described in the literature (**Figure 2B**), which is why it was designated as PD-0 in the following. In more detail, on nucleic acid level there are in total5 nucleotide differences towards the virus PD reported in literature (**Figure 2A**). One of them in 5'UTR and the others in VP1 (one) and VP3 (three). Among the nucleotide differences is one silent mutation in VP1 and another silent mutation in VP3, which have no effect on amino acid sequence. The other two are in VP3 region made differences in two amino acids compared to the state of the art PD sequence. The first amino acid change is at VP3 34 aa. According to the state of PD sequence in bank, which was uploaded by Schmitdke et al, the amino acid at said position is "T", whereas the inventors of the present invention sequences in PD-0 a "M". The last amino acid change is at VP3 237aa. According to the state of the art PD sequence in GenBank, which was uploaded by Schmitdke et al., the amino-acid at said position is "F", whereas the inventors of the present invention sequences in PD-0 a "Y". Whether this single mutation is responsible for all changes the function of PD-0 versus the parental PD is not known. In any case, the inventors could shown that the inventive PD-0 variant can multiply in different target cells and destroy them by cell lysis (**Figure 5**). New viruses are released which, in turn, infect and destroy further cells. At the same time, it is - at the best knowledge of the inventors - assumed that the virus-induced immune mechanisms contribute to the enhancement of tumor cell lysis.

### Example 2: CAR and DAF are differentially expressed on colorectal carcinoma cell lines

Recently, it has been shown that the CVB3 strains Nancy, 31-1-93 and H3 use CAR and DAF for infection, whereas the strain PD uses sulfated Heparan (HS), but may also bind to CAR and DAF. We focused on colorectal cancer as this is one of the most common cancers worldwide with poor outcome and different colorectal cancer cell lines do express HS6ST2, the enzyme, which catalyses the transfer of sulfate groups to position 6 of the N-sulfoglucosamine residue in HS.

Expression of virus receptors on the cell surface is an important prerequisite for virus infection. CAR and DAF represent the primary and the co-receptor for CVB3, respectively. To determine the expression of the different CVB3 receptors on colorectal carcinoma cells, we measured the level of CAR and DAF of nine colorectal carcinoma cell lines (Colo680h, Colo205, DLD1, Colo320, Caco-2, HCT116, SW480, SW620, LS174T) using flow cytometry. Seven cell lines express moderate levels of CAR, while two cell lines (Colo680H and Colo205) express CAR only at a low level. Four cell lines showed high (Colo680, DLD1, Caco2, HCT116) and moderate (Colo320, SW480, SW620, LS174T) levels of DAF expression, whereas one cell line (Colo205) almost completely lacks DAF on the cell surface (**Figure 3A**, **B**). These data demonstrate that CAR and DAF are expressed at variable levels on the cell surface of colorectal carcinoma cell lines, with the majority of cell lines expressing CAR and DAF at high or moderate levels.

### Example 3: CVB3 strains differentially infect and lyse colorectal carcinoma cell lines

To determine whether CAR / DAF expression may influence the ability of CVB3 Nancy, 31-1-93, H3 and PD-0 to infect and lyse colorectal carcinoma cell lines, we used five human colorectal carcinoma cell lines (DLD1, Colo680h, Colo205, Colo320 and LS174T) exhibiting different levels of CAR and DAF expression. These cell lines were infected with a virus strain at an MOI of 1 or 10, and viral infection was determined by measurement of the amount of viral RNA genomes 24 h after infection by quantitative RT-PCR. Each virus strain was detected in its target cell line, but there were significant differences in the infection rates. High and moderate infection rates were detected for PD-0 in all cell lines. Strain 31-1-93 showed also moderate and high infection rates, but only in some of the colorectal carcinoma cell lines (LS174T and DLD1). In the other cell lines, it was low. The infection rates for Nancy and H3 were generally low (data not shown).

To determine cytolytic activity of the CVB3 strains, colorectal carcinoma cell lines were infected with the viruses at a MOI of 1, 10 or 100, and analyzed 24 h, 48 h and 72 h for cytotoxicity using crystal violet staining. The cytolytic efficiency of the CVB3 strains was highly variable. Much like the infection data, PD-0 showed strong cytolytic activity in DLD1, Colo680H and Colo205 and moderate activity in Colo320 and LS174T. The strain 31-1-93 induced cell lysis in four of the five investigated cell lines, but high cytolytic activity was only detected in the cell line DLD1. Nancy and H3 had comparatively low cytolytic activity. Cytolysis was only observed at high MOIs in DLD1 and Colo320 cells. (**Figure 4****, Table 1**). Thus, there was no clear correlation between CAR / DAF expression levels and infection / lysis of colorectal carcinoma cell lines by the CVB3 strains. These data demonstrate that the inventive CVB3 strain PD-0 most efficiently infected and lysed each of the colorectal carcinoma cell lines. It is important to mention that the DLD1 cell line tested here is highly resistant to radiation and against 5-fluorouracil, which is used as chemotherapeutic agent for the treatment of colorectal cancer. Unlike the other CVB3 strains, PD-0 has only low affinity to CAR, but can use N- and 6-O-sulfated HS as receptor to enter cells. The specific receptor tropism to HS therefore seems to be - without being bound by this theory - the most probable explanation for the high infection and lytic capacity of inventive PD-0 in the colorectal carcinoma cell lines. CVB3 viruses according to the present invention may therefore and for said additional features presented be an alternative treatment modality for colorectal cancers, which are resistant to conventional therapies.

**Table 1. CAR and DAF expression and lytic activity of CVB3 strains in different colorectal carcinoma cell lines.**

| **Colorectal Carcinoma Cell Lines** | **Average Expression [%]** | | **Sensitivity of Cell Lines Against CVB3 Variants** | | | |
|---|---|---|---|---|---|---|
| | *CAR* | *DAF* | Nancy | H3 | 31-1-93 | PD-0 |
| DLD 1 | 77,9 ± 14,3 | 93,0 ± 10,8 | ++ | ++ | ++++ | +++++ |
| Colo680H | 7,7 ± 0,9 | 93,8 ± 1,6 | | | ++ | +++ |
| Colo205 | 14,5 ± 8,6 | 1,0 ± 0,1 | | + | + | +++++ |
| Colo320 | 64,6 ± 3,8 | 68,8 ± 6,7 | + | + | + | + |
| LS174T | 52,3 ± 9,3 | 36,1 ± 13,1 | | | | + |

| | | | | | | |
|---|---|---|---|---|---|---|
| Sensitivity was indicated relatively by the number of "+". "+++++" = complete lysis (100%), "++++" = 80% lysed, etc. A free field means resistance of the cell line to the virus variant. | | | | | | |

### Example 4: Heparan sulfate binding mediates oncolytic efficiency of CVB3 PD (proof of concept)

As mentioned before distinct from other strains, PD-0 can use N- and 6-O-sulfated heparan sulfate (HS) to attach and infect cells. To further ascertain the importance of the interaction of PD-0 with HS for infection and lysis of colorectal carcinoma cell lines, the expression level of HS6ST2 was analyzed by quantitative RT-PCR. HS6ST2 catalyses the transfer of sulfate groups to position 6 of the N-sulfoglucosamine residue in HS and leads to production of 6-O-sulfated HS (Hatabe et al., 2013). The investigations were carried out with DLD1, Colo205 and Colo680h cell lines, which are susceptible to PD, and with the LS174T and Colo320 cell lines, which are resistant to PD-0. As shown in Figure 9, HS6ST2 mRNA expression was detected in DLD1, Colo205, Colo680h cells, but not in LS174T and Colo320 cells, indicating a correlation between HS6ST2 expression and susceptibility of colorectal carcinoma cell lines for PD-0.

To confirm that PD-0 indeed uses HS as receptor to infect colorectal carcinoma cells, the soluble HS-analogue heparin was used. CVB3 Nancy was used as control. PD-0 and Nancy were incubated with different concentrations of heparin before infection and virus infection was analyzed by plaque assay 48h later. While infection with Nancy was not affected by heparin treatment and the virus titers were similar at each heparin concentration (Figure 10A), PD-0 infection was distinctly inhibited by heparin treatment in a dose-dependent manner. The effect was more prominent in Colo205 and Colo680h cell lines than in the cell line DLD1 (Figure 10B). Presumably - without being bound by the theory - inhibition of PD-0 infection in DLD1 cell line was lower due to high level of CAR on the cell surface.

The HS blocking experiments were repeated and the cytotoxic activity measured by XTT assay (Roche Diagnostics, Mannheim, Germany). PD-0 induced cell lysis was completely inhibited by 100 µg/ml heparin in Colo205 and Colo680h, whereas inhibition reached about 80 % in DLD1 cell line (Figure 11). The lower heparin-induced inhibition of PD-0 infection and cytotoxicity in DLD1 cells compared to Colo205 and Colo680h cells may be due to high level of CAR on cell surface of these cells, as PD can also use CAR for infection (Zautner et al., 2003).

In conclusion, these data demonstrate that PD-0 as a representative of PD uses HS to infect colorectal cancer cells, and the interaction with HS is a key factor for the cytolytic activity of PD-0.

### Example 5: Construction and characterization of an infectious recombinant full length cDNA copy of avirulent CVB3 strain PD-0 (rPD) inserted into a plasmid

Without being bound by the theory, we hypothesized that a PD clone contains less "mutation variants" after production *in vitro.* Typically, in cell culture amplified PD in general will form a "quasi species" containing several virus "variants" of PD. This may increase the risk of undesirable side effects, e.g. infection of normal cells. Making use of a CVB3-PD-0 clone we expected to reduce this risk. Moreover, standardization of production of PD can be better achieved by use of a PD-0 cDNA clone.

For this approach CVB3 PD-0 full genome was cloned to obtain pure virus stocks of plasmid-based recombinant PD (rPD) and to allow complete genome sequencing analysis. Construction of viral plasmid was done as follows:
Viral RNA of CVB3-PD-0 was isolated by using High Pure Viral RNA Kit, (Roche Diagnostics, Mannheim) according to manufacturer's instructions. Afterwards, reverse transcription was performed with 1 µg of isolated CVB3-PD-0 RNA and reverse primer incorporating a Clal restriction site (CVB3-7381Clal, 5'-GTATCGATTTTTTTTTTTTTTTCCGCACCGAATGCGGAGAATTTA) using 200 U Superscript III reverse transcriptase (Invitrogen) and 20 U RNasin (Promega). After heating at 55 °C for 1 h, then at 70 °C for 15 min, 2 U RNase H was added to the reaction tube and incubated at 37°C for 30 min to remove RNA residues complementary to cDNA. Afterwards, PCR reaction was carried out in 50 ml reaction volumes, containing 2 µl cDNA, 0.5 µl Q5 Polymerase (New England Biolabs), reverse primer which was mentioned above and forward primer incorporating Notl restriction site (CVB3-1Notl, 5'-GGTGCGGCCGCTTAAAACAGCCTGTGGGTTG). Amplification cycles were as follow: one cycle at 98 °C 30 s, 35 cycles at 98 °C for 15 s, 61 °C for 15 s and 72 °C for 5:30 min, terminating with 72 °C for 5 min. PCR reaction directly run on agarose gel and PCR product (approximately 7,400 bp) was cut for gel extraction (Figure 12A). Following gel purification, PCR amplicon was directly inserted to pJET1.2/blunt vector using Clonejet PCR cloning kit (Thermo Fisher Scientific) according to the manufacturer's instructions. Finally control digestion was made following plasmid isolation with Notl and Clal restriction enzymes. Expected band sizes which were 7,5 Kb for full length PD-0 insert and 3.1 Kb for backbone, were observed on agarose gel (Figure 12B). For sequencing Clonejet forward and reverse primer, which spanning the region of insert, were used and samples were sent to Microsynth SeqLab, Berlin, Germany. Sanger sequencing results confirmed successful production of full length recombinant PD-0 clone (rPD) (Figure 12C) with full length PD-0 insert cDNA sequence (Figure 13A), which gives rise to the respective amino acid sequence of Figure 13B.

### Example 6: Construction of high fidelity replication variant (rPD_{HiFi}) of plasmid-based recombinant CVB3 PD-0 (rPD) and subsequent oncolytic activity analysis of rPD and rPD_{HiFi}.

### 6.1 Construction of high fidelity replication variant of recombinant CVB3 strain rPD (rPD_{HiFi})

The positive strand RNA virus replication occurs by RNA-dependent RNA polymerase, which is encoded by viral genome. RNA-dependent RNA polymerase of CVB3 is termed as 3D polymerase. CVB3 can acquire mutations by misincorporation of nucleotides into the viral genome by 3D polymerase. McDonald et al., 2016 found that single amino acid mutation in 3D polymerase protein increased the stability of CVB3 and attenuated the virus growth *in vivo.* Therefore, to increase the genome stability of rPD and to avoid unintended mutation, high fidelity replication variant of rPD (rPD_{HiFi}) was generated. As described by McDonald et al., 2016 phenylalanine which is the 364. amino acid of the 3D polymerase amino acid chain sequence was mutated to tryptophan by site directed mutagenesis using In-Fusion cloning kit (Takara Bio, Japan) with appropriate primers.

### 6.2 Oncolytic activity of CVB3 PD-0 strain variants rPD and rPD_{HiFi} are effectively and lie in the range of wild-type PD (wtPD) in tested human colorectal carcinoma cell lines

T7 transcribed RNA of rPD or rPD_{HiFi} were transfected into the CAR and DAF deficient but heparan sulfate positive CHO-K1 cells for virus production. 48h post transfection cells were administered to three freeze and thaw cycle. Cellular debris was cleared by centrifugation and the supernatant was used for propagation of virus for higher viral titer and to obtain adequate virus stocks. The viruses were propagated in CHO-K1 cell line.

Propagated rPD and rPD_{HiFi} were firstly titrated by plaque assay. Both viruses reached to the similar level of viral titer (around 2 × 10⁷ pfu/ml) (Figure 14). Afterwards, oncolytic activity of rPD and rPD_{HiFi} were evaluated in human colorectal carcinoma cell line DLD1 and murine colorectal carcinoma Colon-26 cell lines. To make a good comparison the wild-type PD (wtPD) and CVB3 H3 were included to the experiment. Results confirmed the retaining oncolytic activity of cDNA derived rPD and also rPD_{HiFi}. Both viruses caused cell lysis both in DLD1 and Colon26 cell lines (Figure 15 A-B). Notably, there were no significant differences between PD-wt and plasmid based rPD or rPD_{HiFi}, assuming that Coxsackievirus B3 strain variants rPD and rPD_{HiFi} are potential and promising anticancer therapeutic virus candidates. However, Colon-26 murine colorectal cell line was found to be susceptible only to PD variants but not CAR dependent CVB3 strain H3 (Figure 15 B).

Based on this finding, we conclude that rPD_{HiFi} grows as good as the parental viruses. Currently we can not say, whether rPD_{HiFi} has indeed lower mutation rates. This is under ongoing investigation. However, given the experimental findings of McDonalds et al., 2016 - at the best knowledge of the inventors - there are reliable lines of evidence to assume that the here constructed rPD_{HiFi} will show lower mutation rates compared to its parental strain, thus which in turn means an increase of the safe use of the virus for therapeutic approaches (safety aspect).

### Example 7: Construction of enhanced green fluorescent protein expressing CVB3 variant rPD (rPD-eGFP)

Reporter genes can be infused to the viral genome to detect or track virus replication. In addition, it also gives an overview about carriage capacity and the stability of the virus in case of exogenous gene insertion. Moreover, demonstrating its functionality when inserted into rPD it is the first step for the development oncolytic rPD which is armed with tumortoxic genes and may have higher oncolytic activity than rPD. Therefore, enhanced green fluorescent protein (eGFP) gene was inserted into the rPD. Firstly, Nrul restriction site incorporated modified 2A pro cleavage site (Lee et al., 2014) was inserted between P1 and P2 polyproteins of CVB3 genome. This plasmid was termed pPD_{2Apro}. EGFP was amplified from pMKS1-eGFP (Tong et al., 2011). pMKS1-eGFP contains full length genome of CVB3 strain H3 and encodes eGFP which was inserted to the downstream of start codon. pPD_{2Apro} was linearized with Nrul enzyme and ligated with amplified fragment encoding eGFP. This final plasmid was termed rPD-eGFP. rPD-eGFP was *in vitro* transcribed using T7 Transcription Kit (Roboklon Gmbh, Berlin, Germany) and RNA was isolated with RNeasy Mini Kit (Qiagen Gmbh, Hilden, Germany). Isolated RNA was transfected into the HEK293T cells and incubated for 48h. Following purification and titration of virus, HeLa and Colon-26 cells were infected to analyze eGFP signal. In parallel, CVB3-eGFP was also used for comparison. HeLa cells, which were infected with rPD-eGFP or CVB3-eGFP, expressed eGFP similarly. However, eGFP signal was only detected in PD-eGFP infected Colon-26 cells but not CVB3-eGFP infected Colon-26 cells (Figure 16). CVB3-eGFP is derived from CVB3 H3 strain, therefore couldn't infect the Colon-26. This result showed that PD-eGFP was still effective to infect H3 resistant Colon-26 similar to previous experiment (Figure 15).

### Example 8: CVB3 strains have strong oncolytic activity in vivo

To evaluate oncolytic efficiency of CVB3 strains *in vivo,* a xenograft BALB/c mouse tumor model was established with the DLD1 human colorectal carcinoma cell line. Tumor cells were inoculated bilaterally into the flanks of the animals and only one of the two tumors was injected with single dose of 3 x 10⁶ PFU of CVB3 Nancy, 31-1-93, or PD-0. Treatment with any of the three viruses resulted in significant suppression of tumor growth of both the virus-injected tumor and the contralateral untreated tumor when compared with uninfected mice tumors (P<0.05; **Figure 5A-**C). Measurement of the amount of infectious virus in the tumors of Nancy and PD-0infected animals by plaque assay 8 and 10 days after virus injection, respectively, showed that both viruses were detected at similar levels (between 3.2 and 7.2 x 10⁶ PFU/g) in the primary injected tumor. Both viruses were also detected in the contralateral, untreated tumor, but the amount was significantly lower in the PD-0 injected group than in Nancy treated animals (P<0.05; **Figure 6A**). Moreover, in two of five investigated animals, PD-0 was not detected in the untreated tumor. *In situ* hybridization confirmed the presence of viral genomic RNA in the tumors of Nancy and PD-0 infected animals, as well as the absence of virus in untreated tumors of two PD-0 infected animals, which also were negative when measured for replicating virus. The inventors also showed that viruses were located in mononuclear immune cells, probably macrophages, within the tumor mass or on the border between the tumor stroma and tumor cells (**Figure 6B**). Taken together, these results demonstrate that CVB3 Nancy, 31-1-93, and PD-0 have strong oncolytic activity *in vivo* and this activity is not only restricted to injected tumor but also is seen in non-injected, distant tumors. Whereas these results demonstrate *per se* the efficacy of CVB3-mediated oncovirotherapy in colorectal cancer, suppression of the untreated tumor reveals the potential of the CVB3-strains to treat metastatic or disseminated colorectal cancer.

### Example 9: CVB3 Nancy and 31-1-93, but not PD-0, induce severe side effects in treated mice

Intratumoral injection of CVB3 Nancy and 31-1-93 resulted in severe side effects. All animals, which were injected with 31-1-93, died six days after intratumoral virus injection and animals treated with Nancy were moribund six (one animal), seven (one animal) and eight days (four animals) after virus administration and were sacrificed. In contrast, the animals treated with PD-0 were alive 10 days after intratumoral virus injection (**Figure 7**).

To elucidate the cause of the severe side effects in the Nancy strain, we investigated occurrence of Nancy and PD-0 infection in heart, liver, brain, spleen, kidney and pancreas, respectively. Using plaque assays to detect replicating virus. The highest virus titers, of about 10⁶ PFU/g, were measured in the heart of the Nancy treated group (**Figure 8A**). CVB3 Nancy was also detected in the spleen, kidney and brain, but at distinctly lower levels, whereas it could only sporadically be detected in the liver (one of six animals) at a very low titer (10² PFU/g). The pancreas is the most susceptible organ for CVB3 in mice (Pinkert et al., 2009). Examination of the pancreas tissue in Nancy infected animals during dissection showed reduced organ size (not shown) indicating major damage to the tissue. As the destruction of the pancreas by CVB3 virus made the tissue unsuitable for determination of infectious virus by plaque assay, we carried out real-time RT-PCR to quantify the virus genomes. High amounts (10³ to 10⁵ copies per µg RNA) of Nancy viral RNA were detected in the pancreas (**Figure 8A**). In sharp contrast, no infectious virus was recovered from any organ of the five PD-0 treated animals. Histological examination of mouse tissues (pancreas, heart, kidney, liver, brain and lung) confirmed different toxic activity of Nancy and PD-0. The exocrine pancreas of Nancy infected animals was nearly completely destroyed and heart tissue of these animals showed distinct inflammation. All other organs of Nancy infected animals were unaffected. In contrast, all organs of the five PD-0 treated animals did not show any alterations (**Figure 8B**). Interestingly, the inventors could show the presence of CVB3 Nancy and CVB3 PD-0 RNA in immune cells within the tumor masses and on the tumor-stroma border zone, whereas tumor cells seemed not to be infected.

Thus, most importantly said animals treated with CVB3 strain PD-0 did not show any virus-induced side effects. No systemic infection with CVB3 PD-0 was detected and virus could not be recovered from the organs of these animals. In addition, PD-0 is nonpathogenic in immune-competent mice. In sharp contrast, two other analyzed CVB3 strains, Nancy and 31-1-93, although showing similar anti-tumor efficiency as PD-0, induced severe systemic infection in mice, leading to the death of the animals. Therefore, these findings reveal PD-0 as an efficient and safe CVB3 strain for treatment of colorectal cancer and a promising candidate for the treatment of cancer, particularly cancer comprising cancer cells which expose and express heparin sulfate receptors.

### References

Fukuhara H, Ino Y, Todo T. Oncolytic virus therapy: A new era of cancer treatment at dawn. Cancer Sci 2016;107:1373-9
Kaufman HL, Kohlhapp FJ, Zloza A. Oncolytic viruses: A new class of immunotherapy drugs. Nature reviews. Drug discovery. 2015;14:642-662
Berry LJ, Au GG, Barry RD, Shafren DR. Potent oncolytic activity of human enteroviruses against human prostate cancer. Prostate 2008;68:577-87
Shafren DR, Au GG, Nguyen T, Newcombe NG, Haley ES, Beagley L, et al. Systemic therapy of malignant human melanoma tumors by a common cold-producing enterovirus, coxsackievirus a21. Clin Can Res 2004;10:53-60
Skelding KA, Barry RD, Shafren DR. Systemic targeting of metastatic human breast tumor xenografts by Coxsackievirus A21. Breast Cancer Res Treat 2009;113:21-30
Miyamoto S, Inoue H, Nakamura T, Yamada M, Sakamoto C, Urata Y, Okazaki T, Marumoto T, Takahashi A, Takayama K, Nakanishi Y, Shimizu H, Tani K. Coxsackievirus b3 is an oncolytic virus with immunostimulatory properties that is active against lung adenocarcinoma. Cancer research. 2012;72:2609-2621
Bergelson JM, Krithivas A, Celi L, Droguett G, Horwitz MS, Wickham T, et al. The murine CAR homolog is a receptor for coxsackie B viruses and adenoviruses. J Virol 1998;72:415-9
Bergelson JM, Mohanty JG, Crowell RL, St John NF, Lublin DM, Finberg RW. Coxsackievirus B3 adapted to growth in RD cells binds to decay-accelerating factor (CD55). J Virol 1995;69:1903-6
Fechner H, Wang X, Wang H, Jansen A, Pauschinger M, Scherubl H, Bergelson JM, Schultheiss HP, Poller W. Trans-complementation of vector replication versus coxsackieadenovirus-receptor overexpression to improve transgene expression in poorly permissive cancer cells. Gene Ther. 2000;7:1954-1968
Stein EA, Pinkett S, Becher PM, Geisler A, Zeichhardt H, Klopfieisch R, Poller W, Tschope C, Lassner D, Fechner H, Kurreck J. Combination of rna interference and virus receptor trap exerts additive antiviral activity in coxsackievirus b3-induced myocarditis in mice. J Infect Dis. 2015;211:613-622
Pinkert S, Westermann D, Wang X, Klingel K, Dorner A, Savvatis K, Grossl T, Krohn S, Tschope C, Zeichhardt H, Kotsch K, Weitmann K, Hoffmann W, Schultheiss HP, Spiller OB, Poller W, Fechner H. Prevention of cardiac dysfunction in acute coxsackievirus b3 cardiomyopathy by inducible expression of a soluble coxsackievirus-adenovirus receptor. Circulation. 2009;120:2358-2366
Andreoletti L, Leveque N, Boulagnon C, Brasselet C, Fornes P. Viral causes of human myocarditis. Archives of cardiovascular diseases. 2009;102:559-568
Andreoletti L, Venteo L, Douche-Aourik F, Canas F, Lorin de la Grandmaison G, Jacques J, Moret H, Jovenin N, Mosnier JF, Matta M, Duband S, Pluot M, Pozzetto B, Bourlet T. Active coxsackieviral b infection is associated with disruption of dystrophin in endomyocardial tissue of patients who died suddenly of acute myocardial infarction. J. Am. Col I. Cardiol. 2007;50:2207-2214
Ronellenfitsch S, Tabatabai J, Bottcher S, Diedrich S, Frommhold D, Schubert-Bast S, Poeschl J, Schnitzler P. First report of a chinese strain of coxsackie b3 virus infection in a newborn in germany in 2011: A case report. Journal of medical case reports. 2014;8:164
Schmidtke M, Merkle I, Klingel K, Hammerschmidt E, Zautner AE, Wutzler P. The viral genetic background determines the outcome of coxsackievirus B3 infection in outbred NMRI mice. J Med Virol 2007;79:1334-42
McDonald, S. Block, A. Beaucourt, S. Moratorio, G. Vignuzzi, M. and Peersen, O.B., (2016). "Design of a Genetically Stable High Fidelity Coxsackievirus B3 Polymerase That Attenuates Virus Growth in Vivo", J Biol Chem, 291: 13999-14011
Tong, L. Lin, L. Zhao, W. Wang, B. Wu, S. Liu, H. Zhong, X. Cui, Y. Gu, H. Zhang, F. and Zhong, Z., (2011). "Destabilization of coxsackievirus b3 genome integrated with enhanced green fluorescent protein gene", Intervirology, 54: 268-275.
Hatabe, S. Kimura, H. Arao, T. Kato, H. Hayashi, H. Nagai, T. Matsumoto, K. M, D.E.V. Fujita, Y. Yamanouchi, G. Fukushima, M. Yamada, Y. Ito, A. Okuno, K. and Nishio, K., (2013). "Overexpression of heparan sulfate 6-O-sulfotransferase-2 in colorectal cancer", Mol Clin Oncol, 1: 845-850.
Zautner, A.E. Korner, U. Henke, A. Badorff, C. and Schmidtke, M., (2003). "Heparan sulfates and coxsackievirus-adenovirus receptor: each one mediates coxsackievirus B3 PD infection", J Virol, 77: 10071-10077.
Lee W., Watters KE., Troupis AT., Reinen NM., Suchy FP., Moyer KL., Frederick RO., Tonelli M., Aceti DJ., Palmenberg AC., Markley JL. (2014).
"Solution structure of the 2A protease from a common cold agent, human rhinovirus C2, strain W12", PLoS One. 2014 Jun 17;9(6):e97198. doi: 10.1371/journal.pone.0097198. eCollection 2014.

## Claims

1. A PD variant of the Coxsackie B3 group (CVB3) virus for use in the treatment of cancer, wherein the PD variant is defined by an amino acid sequence comprising the sequence of Figure 2B (SEQ 2) and wherein the cancer is colorectal carcinoma.

2. The PD variant for use according to any of the preceding claims, wherein the PD variant of the CVB3 virus is defined by a nucleotide sequence comprising the sequence of Figure 2A (SEQ 1).

3. The PD variant for use according to any of the preceding claims, wherein the range of the therapeutically effective amount of the PD variant of the CVB3 virus is between about 5×10⁵ to about 3×10⁸ plaque forming units (PFU).

## Patentansprüche

1. PD-Variante des Coxsackie-Virus der Gruppe B3 (CVB3-Virus) zur Verwendung bei der Behandlung von Krebs, wobei die PD-Variante durch eine Aminosäuresequenz definiert ist, die die Sequenz aus Figur 2B (SEQ 2) umfasst, und wobei der Krebs ein kolorektales Karzinom ist.

2. PD-Variante zur Verwendung gemäß einem der vorhergehenden Ansprüche, wobei die PD-Variante des CVB3-Virus durch eine Nukleotidsequenz definiert ist, die die Sequenz aus Figur 2A (SEQ 1) umfasst.

3. PD-Variante zur Verwendung gemäß einem der vorhergehenden Ansprüche, wobei der Bereich der therapeutisch wirksamen Menge der PD-Variante des CVB3-Virus zwischen circa 5×10⁵ bis circa 3×10⁸ Plaque bildende Einheiten (PFU) beträgt.

## Revendications

1. Variant PD du virus du groupe Coxsackie B3 (CVB3) en vue de l'utilisation dans le traitement du cancer, dans lequel le variant PD est défini par une séquence en aminoacides comprenant la séquence de la figure 2B (SÉQ 2) et dans lequel le cancer est un carcinome colorectal.

2. Variant PD en vue de l'utilisation selon une quelconque des revendications précédentes, dans lequel le variant PD du virus CVB3 est défini par une séquence nucléotidique comprenant la séquence de la figure 2A (SÉQ 1).

3. Variant PD en vue de l'utilisation selon une quelconque des revendications précédentes, dans lequel la gamme du montant thérapeutiquement effectif du variant PD du virus CVB3 est comprise entre environ 5×10⁵ à environ 3×10⁸ d'unités formatrices de plaque (PFU).
